(19) Europäisches Patentamt
European Patent Office
Office européen des brevets

(11) EP 0 667 406 B1

(12) EUROPEAN PATENT SPECIFICATION

(45) Date of publication and mention of the grant of the patent: **21.04.1999 Bulletin 1999/16**

(51) Int Cl.[6]: **D01F 6/06**, D04H 1/42, A61L 2/08

(21) Application number: **95300857.0**

(22) Date of filing: **13.02.1995**

# (54) Process for the production of a gamma-radiation resistant polypropylene fibre for a radiation sterilizable non-woven fabric

Verfahren zur Herstellung einer gammastrahlungsbeständigen Polypropylenfaser für einen mit Strahlung sterilisierbaren Vliesstoff

Procédé de fabrication d'une fibre de polypropylène résistant aux rayons gamma pour tissu non-tissé stérilisable

(84) Designated Contracting States:
**AT BE CH DE DK ES FR GB GR IE IT LI LU MC NL PT SE**

(30) Priority: **11.02.1994 FI 940645**

(43) Date of publication of application:
**16.08.1995 Bulletin 1995/33**

(73) Proprietor: **J.W. SUOMINEN OY**
**SF-29250 Nakkila (FI)**

(72) Inventors:
- **Makipirtti, Simo**
  **SF-29250 Nakkila (FI)**
- **Bergholm, Heikki**
  **SF-00340 Helsinki (FI)**

(74) Representative: **Grew, Eva Regina et al**
**Oy Jalo Ant-Wuorinen Ab**
**Iso Roobertinkatu 4-6-A**
**00120 Helsinki (FI)**

(56) References cited:
**EP-A- 0 198 173**
**EP-A- 0 244 821**
**EP-A- 0 248 545**
**EP-A- 0 303 895**
**EP-A- 0 319 386**
**EP-A- 0 384 382**
**EP-A- 0 391 438**
**EP-A- 0 505 775**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 667 406 B1

## Description

In the process of the invention, fibre is produced from polypropylene polymer using melt spinning methods and, from this fibre, a non-woven fabric is made using primarily hydraulic and/or mechanical and, in special cases, thermal bonding methods. The processing conditions of both the fibre and the fabric production are so adjusted that the sterilization of the end product manufactured from the fabric can be carried out with as little as possible radiation induced damages to the polymer structure.

By choosing the processing conditions in the process of the invention the polymer structure and superstructure of the products are so regulated that the structure in combination with per se known stabilizing agents added to the polymer gives to the products a sufficient stability with respect to gamma-radiation.

The present invention provides a process for the production of gamma-radiation resistant polypropylene fibre suitable for the production of a radiation sterilizable polypropylene non-woven fabric, which process comprises the stages of melt spinning, drawing, finishing, crimping and drying and optionally gamma-irradiating a polypropylene polymer base-stabilized with a peroxide degrading agent and of fibre quality, characterized by:

(a) in a melt spinning stage, controlling the solidification and crystallization of the spinning melt by means of the cooling rate of the melt and the tension of the fibre cable undergoing solidification, so as to obtain a spinning product having, while avoiding a paracrystalline or smectic phase, a structural system with a spherulitic superstructure and a monoclinic-amorphous microstructure, the crystallinity of which is lower than the maximum crystallinity of the polymer and having a crystallinity value higher than 15 mass-%, and an average chain orientation of less than value 0.6 (60%) based on birefringence measurement;

(b) in a solid state drawing stage subsequent to the melt spinning, controlling the super- and microstructure of the polymer of the draw product by regulating the draw ratio and the chain orientation, so as to maintain, while avoiding a microfibrillar structure, the structure of deformed spherulites in the superstructure, and the monoclinic-amorphous system in the microstructure, the crystallinity value being equal to or lower than that of the spinning product and the average orientation being less than 0.76 (76%) based on birefringence measurement

(c) using, in all fibre production stages following the melt spinning and especially in the drawing and drying stages, a temperature not exceeding 120°C for the polymer;

(d) additionally if the fibre is produced at a temperature of 80°C or less stabilizing the fibre against thermal changes by regulating the draw ratio in the drawing stage and, if an irradiation step is present, regulating the gamma-radiation dose;

(e) regulating the tensile strength at yield limit of the product fibre by means of the molecular weight and weight distribution of the polymer, and the draw ratio in the fibre drawing stage; the weight average molecular weight of the polypropylene of fibre quality being between $1.5 \times 10^5$ and $2.5 \times 10^5$, and the molecular weight distribution between 2 and 6;

(f) when the polymer has a high average chain orientation of 0.6 to 0.76, especially close to the area of microfibrillar transition, using at least one radical scavenger or energy extinguisher as a stabilization agent in addition to the basic stabilization.

Preferably the highest actual temperature of the polymer is 100°C.

If the fibre is produced at a temperature of 80°C or less then preferably the elongation of the fibre is regulated by means of the spinning melt temperature, drawing temperature, draw ratio, the molecular weight and weight distribution of the polymer, and radiation dose.

Preferably a temperature of 25 to 80°C is used in the process. Preferably the draw ratio used in the drawing stage is adjusted to be from 1.25 to 4.00.

In one embodiment, the fibre is irradiated with a gamma-radiation dose of from 2.5 to 5.0 Mrad, so that the thermo-mechanical contraction of the product fibre, at applied measuring stress of 0.5 mN/tex, remains below the value of 25% calculated from the elongation of a non-irradiated fibre and simultaneously the strength values of the product fibre remain above the pre- and post-radiation yield limits.

Preferably the gauge of the spinning and draw fibres is from 8.5 to 1.5 dtex.

Preferably the total amount of stabilizing agents present in the final fibre is less than 0.5% by weight of the fibre material.

In the process according to the invention, characteristic features of the fibre and fabric production processes are i.a. the following:

- The spinning and drawing processes operate within a range where the product fibre in the stress-strain system has a definite yield limit before and after irradiation.

- The drawing process subsequent to the melt spinning operates with respect to the polymer chain orientation within a range, where the superstructure of the fibre matrix is formed of deformed spherulites and the formation of a microfibrillar structure is completely prevented. When regulating the primary tensile strength of the fibre by changing the molecular weight of the polymer, the chain orientation can be lowered from the critical range to a suitable value, controlled by the radiation dose and radiation damage, however, remaining within stress and strain areas exceeding the yield limit. It is hereby possible to simultaneously accomplish a regulation of both fibre and fabric elongation.

- With respect to the microstructure of the fibre and fabric product, the process operates within an area where the monoclinic crystalline phase as well as the amorphous phase are stable. The portion of paracrystalline or smectic phase is avoided, especially at high chain orientation. A sufficient amorphous portion in the structure is beneficial from the point of view of process success, irrespective of increased oxygen solubility. In the process according to the invention, the rate and degree of the primary crystallization in the melt spinning process are of importance both for the spinning and in the subsequent partial processes. In practice the regulation generally takes place by means of the simultaneous regulation of the tension of the spinning cable and the amount of cooling air. In addition, the choice of the polymer molecular weight and weight distribution, the control of the quality and amount of additives and adjuvants and catalytic residues (nucleating agents) are of substantial importance for the regulation. The degree of crystallinity determines often together with the temperature and the chain orientation the degree of radiation damage, especially with respect to the tensile strength. On the other hand, the degree of crystallinity has to be adjusted in correspondence with the solubility of the stabilizing agents in the amorphous part of the polymer and to facilitate the even distribution of discharges corresponding to supersaturation by the said agents. As the changes in almost all of the said regulating parameters have a polyfunctional effect, the regulating task is not an easy one.

- In the process of the invention, the radiation dose applied to the fabric in connection with the gamma-irradiation should be big enough to destroy the microbes, but also such that the radiation-induced relaxation of the internal stresses of the fibres is sufficient to prevent and/or regulate the thermal fibre contraction and the corresponding fabric contraction. The magnitude of stress relaxation and contractions as well as fibre and fabric elongation is thus regulated by regulating the degree of average fibre chain orientation as well as radiation dose, the minimum radiation dose being appr. 2.5 Mrad.

- In the process, the desired fabric strength is achieved by regulating the fibre yield limit energy at stress by varying the molecular weight and/or the chain orientation of the fibre polymer. In the regulation of the fabric strength, use is hereby made of the empirical interdependence between the yield limit energy and the water jet needling energy. It is especially to be noted that the fabric radiation damage in question is also dependant on the fabric structure and often less than the corresponding fibre damage. The fibre radiation damage affects the fabric elongation, especially at low chain orientation values.

- In the process according to the invention, specific temperature limits are required of the various partial processes included in the fibre production as well as in the production of water needled fabrics, which temperatures cannot be exceeded without damaging the products manufactured from the fabric during gamma-sterilization. The same temperature limit requirement applies also to post-treatment processes of the products made from the fabric (e.g. dyeing, hydrophobicity and heat treatments). Experimentally it has been established that the total radiation damage is the result of a thermally activated mechanism, whereby the activation energy is of the order of $E = 40$ kJ/mole. Apparently a polymer structure induced damage is involved, appearing already during the gamma-excitation, as it has been established that the so called storage damage to its thermal activation primarily corresponds to that of hydroperoxide degradation. According to measurements, radiation damage with respect to elongation can be detected already at the glass transition temperature and in the area of stress orientation it reaches the value 85% at a temperature of appr. 120°C. Naturally many other factors affect the damage besides the temperature. The said temperatures are the actual structural temperatures, which are measured (independently of the so-called machine temperatures) from the long identity period values of the polymer, which values, under the said conditions, are monotonic functions of the temperature.

The process according to the invention comprises technically highly developed improvements in per se known fibre and fabric production processes, using additives to be compounded into the polymers to lower the side-effects of high-energy radiation.

In the following, the present state of the art is discussed relating to fabric water needling methods, fibre melt spinning methods and prevention of polyolefin gamma-radiation damage.

In the water needling method, the fibre material is treated with water. Water is forced under high pressure through dies arranged in sequence in straight or staggered rows to form very thin liquid jets. The fibre material forms a layer wherein the individual threads, fibres or staple fibres retain their identity and freedom of movement (non-woven). The fibre material layer is transported on a conveyor and processing support made from a perforated sheet or wire with respect to the said die row, whereby the high energy liquid jets entangle and mix the fibres in a manner which increases the strength and the stability of the fibre layer without the presence of a separate binder.

Figure 1 shows schematically a water needling production line, which after pilot-scale test series was used for carrying out the method /1/. The parts of the apparatus are as follows: 1. fibre feeder, 2. carding apparatus, 3. and 4. water needling stations, 5. dryer and 6. fabric winding.

Figure 2 shows schematically a mode of operation of one die row in the water needling station. The parts included in the scheme are /1/: 1. distribution beam with dies, 2. jet row, 3. web, 4. wire, 5. honeycomb casing, 6. vacuum slits with insulation, 7. vacuum chamber and 8. process water.

After the pioneer inventions relating to hydraulic aperturation methods (USP 2.862.251/1958, USP 3.033.721 and USP 3.081.515) work was started on a large scale for the development of liquid needling methods as well as their productional applications. Among patents from this stage, the methods of USP 3.485.706/1969, USP 3.485.708 and USP 3.508.308 may be mentioned. There is a very large number of patented apparatus, method and product inventions which are modifications or improvements of these central inventions, i.a. /1/.

The process according to the invention is as regards the production of propylene fibre not bound to any particular production apparatus. Both in pilot and in production scale test runs, a melt spinning apparatus with a short cooling section, and a conventional drawing apparatus were used. The pilot apparatus was also of production scale as regards the melt spinning section.

Figure 3 shows schematically the spinning-drawing apparatus used in the tests. The various parts of the apparatus shown in the diagram are: 1. extruder, 2. melt spinning device, 3. 1-godet, 4. drawing oven, 5. 2-godet, 6. 2-avivage, 7. crimping device, 8. stabilizing and drying oven and 9. staple fibre cutter.

The known /2/, quite high present technical level and knowledge relating to the various fibre production stages will not be discussed in this connection.

Gamma-irradiation is in fairly wide use for the sterilization of medical apparatuses and devices (cloths and cloaks for surgery, wound dressings, syringes) *i.a.* in ready-made packages prior to their storage in aseptic conditions. The minimum dose, 2.5 Mrad, conventionally used in sterilization, is sufficient for the destruction of the common microbes. This radiation dose, however, causes strong degradation in polypropylene, which as a result of various thermal and autooxidative chain reactions continues effectively during post-irradiation storage.

The gamma-radiation induced polymer degradation is primarily a peroxide- and alkyl radical-mediated chain oxidation process. The process can be illustrated /3, 4, 5/ with the following simple reaction mechanism formula (PPH - polypropylene, $PP^{\bullet}$ - free radical, $PPO_2^{\bullet}$ - peroxide radical, PPOOH - hydroperoxide):

1 $PPH + \gamma \rightarrow$ [caged macro-radic.pair] $\rightarrow PP^{\bullet}$ initiation

2 $PP^{\bullet} + O_2 \rightarrow PPO_2^{\bullet}$

3 $PPO_2^{\bullet} + PPH \rightarrow PPOOH + PP^{\bullet}$ } propagation

4 $2PPO_2^{\bullet} \rightarrow$ no radic. products termination

for example 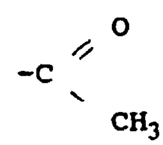

Into the formulas also a terminal re-initiation may be included ("X" - reactive center, for example metal ion):

$$5 \quad \left.\begin{matrix} PPOOH \\ PPOOH+"X" \end{matrix}\right\} \begin{matrix} \text{different} \\ \text{degradation} \\ \text{mechanisms} \end{matrix} \rightarrow \begin{bmatrix} PPO^{\bullet}, {}^{\bullet}OH \\ PPO_2^{\bullet} \end{bmatrix} + PPH \rightarrow PP^{\bullet}$$

Of the excited states and radicals induced by the gamma-radiation energy (reaction 1.), the dominating alkyl radical is

$$(CH_2 \cdot \underset{\underset{CH_3}{|}}{C} \cdot CH_2-)$$

which is present evenly distributed in the amorphous and crystalline phase of the polymer. Under the influence of oxygen diffusing into the amorphous part of the polymer, the radicals are oxidized (almost without thermal activation) and as reaction products, *i.a.* carboxylic acids, alcohols, and different peroxides are obtained (reactions 2-3). In the termination reaction (reaction 4), a chain terminating ketone is the main product as a result of breaking up of the main chain. The degradation of peroxides (*i.a.* reaction 5) provides for a tremendous increase in the number of radicals through a chain mechanism. This degradation is a thermally induced, low activation process. Degradation is thus time and temperature dependent, the oxidation reactions comprising also effects of radiation dose rate, temperature and shelf-ageing. According to present knowledge, the slow, thermally activated degradation of hydroperoxides (reaction 5.) results in shelf-ageing /4, 3/. The strong radiation-temperature-synergism (PE:/4/) present in the simultaneous or consecutive radiation oxidation (in air) is mediated by the thermally induced degradation of primary hydroperoxides initially formed by irradiation.

The damages caused by radiation oxidation of polypropylene can be reduced by suitable stabilization agents or their often synergistic mixtures added to the polymer. Due to the production technology and modes of application of both the fibres and the corresponding fabrics, the area of the invention under consideration includes, besides high-energy radiation, other conventional energy types, such as heat and light energies, and mechanical energy. Due to the diversified need for protection there is a considerable number of polypropylene stabilizing agents and modes of performance thereof.

Stabilizing agents and their mixtures used in the method according to the invention are discussed in a general manner /6/.

Some organic compounds have the ability to terminate the chain oxidation reaction of a polymer so that a fast reaction between such an inhibitor and free radicals of the polymer takes place, giving as a product a free radical which is more stable than the original, and an inactive polymer chain. The free radical is not sufficiently reactive with respect to molecular oxygen and thus does not promote the chain reaction, but may be deactivated and regenerated to form the original inhibitor molecule. To these primary antioxidants (that is chain terminators or free radical scavengers) belong *i.a.* the hindered phenols, penta- and tetramethylpiperidines and corresponding nitroxides.

When hydroperoxides degrade to form free radicals (and the oxidation cycles continue autogenously: mechanism reaction 5) they absorb the required energy of conversion from the surrounding in the form of light and heat. The initiation rate of this thermal and photooxidation can be reduced by degrading the peroxides to non-radical components before their degradation. Many organic compounds are able to degrade peroxides. Such peroxide degrading agents, termed secondary antioxidants, are *i.a.* various sulphur, phosphorus and nitrogen compounds, secondary and tertiary amine compounds, and hindered amines.

A potential means of protecting the polymer from oxidative degradation is to prevent the excitation energy from absorbing therein. Absorption of UV-radiation energy is a conventional protection against initiation of photooxidation. Hereby the absorbed energy is released at an energy level lower than the original. Hydroxybenzophenone and benzotriazole derivatives may be mentioned as UV-stabilizing compounds.

Excitation of the various types of chromophores of polypropylene produces excited states leading to initiation reactions. One release path for a chromophore is therefor the transfer of its energy to a stabilizing agent, which is also possible only when the acceptor energy balance is below that of the donor. Such energy extinguishers-stabilizers are i.a. the sterically hindered phenols, penta- and tetramethylpiperidines.

In the method according to the invention the group consisting of hindered amines /3/ was used as a primary polypropylene stabilizer, which amines in addition to the group of radical scavengers, are included both in the groups of peroxide degrading agents and energy extinguishers. A synergist to the hindered amines was the group of benzophenones known as UV-absorbants. The basic polymer stabilizing mixture was the phosphite-phosphonite-mixture belonging to the group of peroxide degrading agents and calcium stearate. The structure and names of the stabilizing

agents used in the method are disclosed in Table 1. It may be mentioned that the chain reaction between the free radicals corresponding to the gamma-excited chain oxidation reactions 2. and 3., and a hindered amine (in the method, a tetramethylpiperidine compound was used), can be illustrated /3, 9/ with the reactions 6-8.

6 $\rangle NH \rightarrow \rangle NO^{\bullet}$

7 $\rangle NO^{\bullet} + PP^{\bullet} \rightarrow \rangle NOPP$ or $\rangle NOH$

8 $\rangle NOPP + PPO_2^{\bullet} \rightarrow \rangle NO^{\bullet} + PPOOPP$

Thus the amine is first oxidized to form a stable nitroxyl radical ($\rangle NO^{\bullet}$), which scavenges the macroalkyl radical ($PP^{\bullet}$) forming a substituted hydroxyl amine ($\rangle NOPP$) which in turn reacts with the peroxide radical forming a reactive nitroxyl radical.

As examples of the state of the art relating to the development and use of stabilizing agents for polymers for decreasing the negative effects of energy-rich radiation, the following patent publications may be mentioned /7/:

Patent GB 1.050.802 discloses a stabilizing system for polyolefines, wherein an organic phosphite and a carboxylic acid convert the structure of a polymer matrix of a moulded body (such as a medical syringe) to be composed of spherulites, the diameter of which is less than 10 µm.

The patent series USP 4.110.185, 4.274.932, 4.467.065 comprises a stabilizing system wherein a mobilizing agent (hydrocarbon, polymer wax) is added to a semicrystalline polymer having a narrow molecular weight distribution to increase its free volume. As a result of the increased mobilization of the amorphous portion, the radical termination rates increase, whereby the polymer (for example medical syringes, and films) chain oxidation during and after irradiation substantially decreases. The termination rate measurements of the inventors support the patent specifications /8/.

The patent USP 4.563.259 comprises a polyolefin stabilizing method, wherein, in addition to a liquid mobilizing agent corresponding to the afore mentioned patent series, a heterocyclic hindered amine (the said 2,2,4,4-tetramethylpiperidine derivatives) is added to a polymer having a narrow molecular weight distribution range.

The relationships between polymer characteristics, production factors and gamma-excited chain oxidation will be discussed shortly. In the technical scientific literature this is very poorly known.

The effect of the polymer characteristics on its oxidation rate /10, 11/ may be listed as follows:

Molecular weight: When the molecular weight increases the initiation rate of radical formation decreases, but simultaneously the decrease in the termination rate compensates for the effect, wherefore the overall oxidation rate does not substantially change.

Tacticity: A stereoregular chain system is more resistant to oxidation than an atactic system. In an isotactic system, the oxidation rate may be a function of the amount of the atactic component.

Contaminants: Polymer additives, catalyst residues, metal containing contaminants, and stacked oxidation products usually increase the oxidation rate and often have even an autocatalytic effect thereon.

Material thickness: The effect of the material thickness on the oxidation rate is a function of the difference between the oxidation reaction and oxidation diffusion rates. Different values between 8-250 µm have been given for the limit thickness for a film or fibre sample /10, 11, 12, 13/.

Morphology and material transport: The mobilities and solubilities of the components participating in the gamma-initiated oxidation reactions are very important in technical oxidation processes. The values of the mobility determining diffusion as well as the solubility coefficients, corresponding to the various states of the polymer matrix, are very poorly known. Seen from a radiation oxidation point of view, the mobility of material is considerable only in the amorphous portion of the polymer, and also in defective crystalline morphology (paracrystalline). The solubility of molecular oxygen and hydrogen and also of stabilizers and other additives is in practice limited only to the amorphous portion of the polymer, where the free volume is sufficient /14, 15/. Besides an increase in crystallinity, an increase in the chain orientation of the amorphous portion of the olefin polymer (decrease in the free volume) as well as changes in the superstructure reduce (at the order of a decade) especially the oxygen and stabilizer solubility and mobility /16, 17/.

The values of inter- and self-diffusion of the polypropylene chains and their parts, radicals and oxidation products are unknown, but they can be estimated using various models of calculation /18/.

As an example of the diffusion rate and activation energy in polypropylene, the following values may be given (D/25°, $cm^2/s$: E, kJ/mol: spherulitic matrix, no crystallinity correction made, /14, 11, 19/)
oxygen: $D = 3.59 \times 10^{-7}$, $E = 46.1$; hydrogen: $D = 2.12 \times 10^{-6}$, $E = 34.7$;
TV 770 (Table 1): $D = 1.17 \times 10^{-11}$, $E = 95.6$.

Finally two publications are considered from the point of view of the effect of production conditions on the oxidation of polypropylene: The effect of the production conditions on the photosensibility of polypropylene monofilaments and films has been studied /13/ by measuring the stacking of hydroperoxides and carbonyls in the polypropylene matrix as a result of a Xe-beam-excited and partly also (independent of chromophore and oxidation product stacking) gamma-excited oxidation.
The measurement results give rise to the following conclusions:

- The oxidation of filaments and films (gamma-initiation) was independent of the morphological differences between the samples in the area under study.
- As there was no effect due to morphological differences, the differences in light sensitivity (UV-initiation) are apparently due to differences in the chromophore concentrations.
- Chromophore production resulting from polymer bond scission takes place in the drawing stage of the filament, specially at low draw temperatures and high draw rates. The amounts of oxidation products do not, however, correlate with filament brittleness.

In the method according to the Finnish lay-open publication 88804, the effect of some production factors on the radiation resistance of polypropylene are disclosed. According to the method, a radiation-resistant product is made by treating the polymer melt and the extrudate obtained therefrom so that the proportion of the smectic phase is high (*i.e.* about 40-75 mass %) and the proportion of the monoclinic crystal phase is less than about 20 mass %. According to the method, when changing the dimensions of the extrudate, the final draw ratio must not exceed the value $\lambda = 1.50$, and preferably the draw ratio should be less than the value $\lambda = 1.10$. The polymer chain distribution is in the range of $M_W/M_n \cong 3\text{-}6$.
Of the method the following can be said briefly:

- In the method, known strongly radiation-stabilizing agents have been used, the effect of which does not differentiate from the said method.
- The method concerns rapid spinning methods, whereby the product generally has primarily a so-called smectic spinning structure.
- The phase ratios used in the description of the method and in the claims are confusing (one phase is missing) as well as misleading.
- The long polypropylene identity period is not in a monotone relationship to the proportions of the monoclinic or smectic phase, and also not to the radiation damage.
- The description of the method is insufficient for the inambiguous understanding of the invention, and it does not support the claims.

Based on the evaluation of the state of the art, gamma-radiation induced structural damage in polypropylene is to its mechanism very complicated and to many parts also still qualitatively unsolved.
In order to illustrate the differences between the basic features of the invention already disclosed and the known state of the art, examples will be employed to examen essential observations and test results relating to the method.

Example 1.

In the example 1., the basic principles for the invention and the essential observations relating thereto are shown by means of detailed subexamples.

Subexample 1.1

In the subexample 1.1, the functional equations for the water needling and fibre production apparatuses used in the process development and corresponding to the exemplary part of the invention, are shown.
In the water needling test series on a pilot scale, a needling station analogous to a production apparatus was used (Fig. 2), where the needling took place as a surface needling. The web-wire-honeycomb system (Fig. 2: 3, 4, 5) of the needling station is made movable in two directions at adjustable speed with respect to the nozzle bar (Fig. 2: 1, 2) and the suction device (Fig. 2: 6, 7).
The strength of water needled fabrics is proportional to the water jet energy received by the web, that is proportional also to the jet energy developed by the apparatus. From the experimentally measured jet scattering values due to nozzle speed and disturbance factors, the nozzle effect ($P_L$, w/m$^2$) as a function of the pressure difference ($\Delta P$, bar) and nozzle distance (L, mm) per unit width of the nozzle bar (m) is obtained

$$P_L = 14.70 \times 10^5 \times (\Delta P)^{1.068} \times L^{-0.5}. \quad /1/$$

By introducing the number of nozzles (1557 per m), the area of the nozzle opening (0.127 mm) and the speed of the web ($v_r$, m/s) into the equation, one obtains a value for the jet energy (w, ws/$m^2$) directed against the fabric per unit area ($m^2$) of the fabric

$$w = 28.99\, [\Delta P]^{1.068}\, L^{-0.5}\, v_r^{-1.0}. \quad /2/$$

The jets having released their energy for the fibre "mixing-bonding", the amount of water brought to the needling location has to be continuously removed with the suction-vacuum-apparatus in order to eliminate any jet energy losses in subsequent needling operations.

Fig. 4 shows values obtained on a pilot apparatus of the tensile strength of a water needled fabric as a function of the energy sum calculated from the afore mentioned equations.

The tests in the series were carried out using a series of constant pressure as a function of increased wire speed, and using a constant wire speed as a function of a series of increased pressure, and consequently the energy sum was formed in two ways. In the test area studied, when needling a polyester web, the results of both the pressure and the speed series are the same. The pressure series are for both the polyester and the polypropylene web series almost the same, but the values of the speed series are for polypropylene somewhat lower than those for polyesteer (the mutual differences are functions of the physical characteristics of the fibres, and avivage differences). In the test series used for the method, a wire speed of $v_r$ = 10.9 m/min was used. In the pre-needling of the web, the pressure series of p = 30-60-80 bar was used and the wire size 92 x 100 mesh, and in the final needling the pressure series of p = 30-60-100 bar and the wire size 22 x 24 mesh. When using these test series, the said measurements give matching fabric strength and energy values both in the pressure and the speed series.

For the production of the fibre sample series used in the description of the method according to the invention, the pilot scale spinning-drawing-apparatus shown in Figure 3 was used. The number of nozzles in the nozzle plate of the spinning device was 30500 and the nozzle diameter 0.25 mm. According to measurements carried out, the capacity of the apparatus (P, kg/h) was a function of the rotational speed of the pump ($n_r$, $min^{-1}$) of the form

$$P = 2.0437 \times n_r. \quad /3/$$

The velocity of the polymer in the nozzle is correspondingly of the form
($v_s$, m $min^{-1}$)

$$v_s = 2.5111 \times 10^{-2} \times n_r. \quad /4/$$

In the measurements carried out for the method usually a rotational speed of the polymer pump of $n_r$ = 15.15 was used, whereby the spinning capacity and the nozzle speed were correspondingly:

$$P = 30.96 \text{ and } v_s = 0.3804.$$

In the apparatus shown in Figure 3, the speed of the 2-godet and the fibre gauge ($v_2$ and $d_2$: Fig. 3: 5) were usually kept constant in the test series ($v_2$ = 90 and $d_2$ 2.2 or 1.7). In some cases the speed of the 1-godet of the apparatus was kept constant, whereby at constant product gauge, the capacity of the apparatus varied.

Subexample 1.2

In the subexample 1.2, the relationship between the water needled fabric strength and the corresponding fibre strength is shown.

It is difficult to define a detailed form for the relationship between the strength characteristics of water needled fabrics and corresponding fibres, because the "mixing-bonding" of the fibres as a result of the water needling process comprises almost all possible forms of fibre deformation.

In this study use is made of the observation that when the other fabric production parameters remain constant,

its tensile strength is almost a linear function of the yield limit energy of the corresponding fibre. It is self-evident that also other relationships can be used. The tensile strength - yield energy relationship is shown in the pilot test series no. 057. In the test series, the polypropylene polymer I is used in the samples no. 1-8 and polypropylene polymer II in the samples no. 9-22. The MWD and MFI values for these polymers are given in Table 2. The production parameters for the fibre are indicated in Table 3. The numbering of the processing stages indicated in Table 3 corresponds to the numbers in Figure 3. The tensile strength ($\sigma_{11}$, N/5 cm) of fabric samples made from the fibres of test series no. 057, as a function of the fibre yield energy (Iy, ws), is indicated in Figure 5. The equation of the line drawn through the measurement points has the form

$$\sigma_{11} = 1.459 \times 10^5 \text{ Iy} + 58.61. \qquad /5/$$

According to the figure, the tensile strength-yield energy-function is sufficiently linear over a large area and is thus applicable for evaluation and comparison of the method being studied. It is to be noted that the position of the function in the diagram is dependant *i.a.* of the used fibre polymer, the structure of the water needled fabric. In the production of the fabrics of the example, the conditions indicated in the subexample 1.1 as regards speed, pressure and wire series, were used.

Subexample 1.3

The subexample 1.3 shows the nature of the radiation damage of the polypropylene fibre in the stress-strain-function, which is of importance for the properties of the water needled fabrics.

In order to study the stress-strain relationships, the pilot test series no 82 A and 82 C were made. The production conditions for the test series as well as the polymer used have been indicated in the Tables 2 and 3. In the Figure 6, the stress-strain curves corresponding to the fibres of the test series no. 82 A are indicated. The fibre sample corresponding to the draw ratio $\lambda = 2$ (no. 3) has been used as the reference. The stress-strain-values for the samples corresponding to further draw ratios ( $\lambda$: 1.25, 1.50, 2.50 and 3.00) have been multiplied with a coefficient obtainable from the values at break for the samples (Fig. 6), to correspond to the reference values (at their final states). In the Fig. 6, the stress-strain-values for the gamma-irradiated samples of the test series no. 82 A (dose: 131 krad/h *i.e.* 3.15 Mrad, storage time 14 days, marked s) have been indicated, using the above mentioned coefficients.

According to the Fig. 6, the radiation induced embrittlement of the fibre structure appears under stress-strain conditions exceeding the yield limit. At the yield limit, at still almost completely recovering spherulite structures, the shear forces and a corresponding lamellar gliding dominate. In the yield area the spherulites are stretched, however, maintaining the intraspherulitic continuity. The transition in this area takes place as processes of lamellar gliding, increase in lamellar distance and orientation. At the beginning of the next, that is the processing-consolidation area, the transition from deformed spherulite to microfibrillar structure is already complete. The additional increase of the fibre elongation in this area leads to a continuous increase in strain up to the break limit. These stress-strain areas with their changes as functions of the draw ratio (deformation) are clearly apparent from the Fig. 6.

The fibres of the test series no. 82 A have been drawn at low machine temperature (80°C) and are thus unstabilized for other reasons. The draw structure of the sample fibres is thus completely unrelaxed and quite far from the thermodynamic equilibrium. *I.a.* the tie chains which interconnect the crystals, are thus under especially high interior stress, the magnitude and quality of which are related to the magnitude and corresponding structure of the deformation (draw ratio) applied to the fibre.

According to measurements, the border area between the spherulite and microfibrillar polymer structures is reached at an average chain orientation value range of fav = 0.70 - 0.75. According to birefringence measurements, the interrelationship between the average orientation and the fibre draw ratio for the series no. 82 A fibres, in the area under examination, can be expressed in the form: fav = 0.1525 $\lambda$+ 0.4125. Thus the fibre samples no. 4 and 5 are to their structure already microfibrillar, whereby in the system, in addition to radiation-induced radicals, there are present also considerable numbers of work-induced radicals. In this case, the degradation of the chain structure is more rapid than usual. The decrease of the strength values of the samples of the draw series 82 A appears in the stress-strain-area above the yield limit at the radiation dose used, whereby a change in tensile strength corresponding also to big elongation changes, is small.

Based on the radiation behaviour of the fibres it is expected that in water needled fabrics only small changes with respect to tensile strength and elongation take place at the radiation doses tested. This can be seen from Fig. 5. The figure shows the yield energy-tensile strength relationships of the test series 057 fibres and corresponding fabrics (dose 3.15 Mrad, storage 40 days; samples no. 18-22, 10-12 and 14-15).

The strength behaviour of the fibres of the test series no. 82 A at elevated temperature before and after

irradiation (3.15 Mrad + 84 days) was studied using thermo-mechanical analysis. The fibres to be studied were stressed (0.5 mN/tex) and their change in length was studied as a function of temperature, at increasing temperature (10 °C/min).

In the Fig. 7 the general behaviour of TMA-elongation for the test series fibres no. 3 and no. 5 ( $\lambda$= 2.0 and 3.0) is seen. According to the figure, the high contraction maximum for the sample 5 (54.3%/169.3 °C) decreases substantially under the influence of radiation (24.4%/161 °C). The contraction peak for sample 3 (37.5%/167 °C) is changed into elongation under the influence of radiation. The strong decline of the contraction peaks and the corresponding "flow"-temperatures of the polymers as a result of radiation, is also considerable.

The Fig. 8 shows the values of the TMA-contraction peaks as a function of the fibre draw ratio for the samples no.82A of the test series, the heat treated (120 °C/30 min) samples of the same series and for the samples of the test series 82 C produced at an increased 2-godet temperature (machine temperature 145 °C). The figure shows the strong effect of radiation on the length contraction of the samples, especially at high draw ratios.

Based on thermomechanical measurements, it can be established that at the very low radiation doses used, the partial gelification of the polymer matrix has a relaxing effect, corresponding to that of thermal stabilization, on the stress states of the fibre structure, thus eliminating the detrimental effects thereof. The stabilization of the structure is also evident from the decrease in the first order reflection intensity in small-angle X-ray diffraction measured from the samples of the test series 82 A, under the effect of radiation. Irradiation of the fibre polymer is followed by a slight increase in the degree of crystallinity, but as the additional crystallization takes place as parallel and chain terminal crystallization in the amorphous areas, the result is a decrease in the electron density in the lamellar system (the long identity period remaining constant). This result is quite important, as the magnitude of stabilizing heat treatment of both the fibres and the product fabric can be maintained low, as also the temperature favouring radiation damage. It is also to be especially noted that the said radiation stabilization to its magnitude can be quite precisely controlled and regulated, for example by means of the fibre draw ratio and the radiation dosage.

Subexample 1.4

In the subexample 1.4, the behaviour of polypropylene fibres, manufactured under production conditions, during irradiation and especially during the subsequent storage, is shown. In the irradiation study, the fibres of the test series 82 A were used. In order to reduce radiation damage, the components Tinuvin 770 and Chimassorb 81 were added as stabilizers to the fibre polymer so that the concentration of each component in the end product was 0.15 % by weight. The structural formulas of the stabilizing agents are shown in the Table 1. The basic stabilizer of the polymer was the phosphite-phosphonite mixture mentioned in the said table.

For production technical and structural reasons the fibres obtained from the 1-godet from melt spinning (Fig. 3: 3) and the actual product fibres (that is the fibres from the 2-godet, from crimping and fixation) are studied separately in the following.

The average orientation factor of the spinning fibre is a function of the spinning draw ratio ($\lambda_k$). The orientation factor on the other hand, is defined as the function

$$f = \Delta n/\Delta n_{max} = ½(3 \cos^2\theta - 1)$$

wherein $\Theta$ is the angle between the draw direction and the structural main axis, and $\Delta n$ is the refractive index difference. The orientation factor for the spinning fibre samples can be given the form, as a function of the spinning draw ratio,

$$fav = 0.145 \ln \lambda_k - 0.217. \qquad /6/$$

The elongation value for the spinning fibre ($\varepsilon_o$, %) is, in the area under study, a linear function of the orientation factor, of the form

$$\varepsilon_o = a_i \, fav + b_i \qquad /7/$$

Spinning fibre samples were irraditated for one day with gamma-radiation, the dose being 132 krad/h. After irradiation it could be established that the orientation factor was unchanged within the accuracy of measurement. Also the elongation maintained its function form in the postradiation conditions (/7/). The decrease in elongation of the spinning fibre samples as a function of storage time was rather low. The angular coefficient of the elongation function maintained its value as a function of time, and the elongation was of the form

$$\varepsilon_s = a_i \, fav + ct^{-n} \qquad /8/$$

The storage time varied between 14 to 805 days. The time dependency of the function can advantageously also be presented as a logarithmic function of the storage time.

The standard coefficients of the elongation function of the spinning fibre samples before radiation and as a function of the postradiation storage time, are given in the Table 4. The spinning elongations before radiation and corresponding to a storage time of 271 days (as an example) are given as a function of the orientation factor in the Table 5. In the Figure 9, the initial values for the elongation as well as some elongation values corresponding to a number of post-radiation storage times, are indicated. These values have been shifted by means of the coefficient obtainable from equation 8 to correspond to a storage time of 44 days. From the figure it can be seen that the measurement results for the sample fibres (14, 44, 130, 271, 443, 805 days) satisfactorily conform to the orientation and time equations (6, 7 and 8).

The orientation factor for the fibre samples obtainable from the 2-godet (Fig. 3: 5) of the test series no. 82 A can, in the area under study, be expressed as a function of the draw ratio in the form

$$fav = 0.158\lambda + 0.398 \qquad /9/$$

The elongation value for the drawn fibre is, in the area under study, a decreasing linear function of the orientation factor in analogy with the form of the equation 7. It is divided into two linear parts when the limit value for the orientation factor is: fav = 0.76. The values of the standard coefficients of the functions have been indicated in the Table 4 and the measured elongation values ($\varepsilon_o$) in the Table 5.

The function form of the orientation-elongation changes is maintained also in the samples corresponding to the post-radiation storage times. As an example of this, the measured results corresponding to a storage time of 194 days have been indicated in the Tables 4 and 5.

Within the limits of accuracy of measurement, the elongation functions of fibre samples of the test series from the 2-godet, corresponding to different post-radiation storage times, intersect in the same point having the coordinates $\varepsilon_s$ = 115 % and fav = 0.76. For very long storage times, the elongation values in the area fav > 0.76 shift to elongation values lower than the said value, the polymer matrix at the same time degrading rapidly and the apparent linearity of the functions disappearing. The storage time, the stabilizing agents and stabilizing method, the polymer MWD and other factors have an effect on the magnitude of the limit elongation. The dependency of the elongation function on the storage time is small, and can be expressed analogously to the function according to the equation 8. The standard coefficients for this function for both orientation ranges are indicated in the Table 4.

In the Figure 10, the elongations of the 2-godet samples of the test series no. 82 A before and after irradiation, have been indicated. The storage time of 14 days has been taken as the reference. The measurements corresponding to other storage times have been shifted to correspond to a reference time by means of a coefficient obtainable from the time equation: [115+F(t,14)(0.76-fav)]/[115+F(t,x)(0.76-fav)]. According to the figure, the measuring results fit well the equations simulating them. In the area of high orientation, the results show a considerable spread for long storage times.

In the Figure 11, the decrease of the radiation induced fibre elongation, $-\Delta(\varepsilon) = (\varepsilon_o - \varepsilon_s)/\varepsilon_o$ as a function of the average orientation factor is shown. The reference time is 14 days (after irradiation). The figure shows the surprising decrease of the radiation damage ($-\Delta\varepsilon$) (also for long storage times) when the orientation increases in the area; fav < 0.76. This phenomenon takes place in the area of deformed spherulite polymer structure, and a cause for this can at least partly be seen in the decreased mobility of oxygen and also of radicals resulting from this polymer structure and also from increased orientation. Besides the structure, also the number of mechanically generated radicals promote the considerable radiation damage in the microfibrillar area (fav > 0.76).

Subexample 1.5

In the subexample 1.5, the gamma-radiation induced changes in the stress-strain properties of a water-needled fabric series and the corresponding fibre series are shown. In the short test series of the subexample, sample series of the fibre test series no. 109 were used. In the sample series 109A, 109B and 109D the polymer II and in the series 109F the polymer III (Table 2) was used. In the sample series 109A, 109B and 109F an addition of stabilizing agent was used: 2.7 % by weight of polymer VII, 0.15 % by weight of hindered amine Tinuvin 770 and 0.15 % by weight of technical benzophenon (Table 1). In the comparative sample series 109D, no stabilizing agent addition was used. The fibre gauge and correspondingly the feeding capacity (d =2.2 dtex and $n_r$ = 15.15 $min^{-1}$) of the fibre sample series 109A were higher than the values for the other sample series. The test conditions of the test series (109) (Table 3)

were in other respects comparable. The fabric samples were produced in the manner described in the subexample 1.1.

In order to show the changes in the stress-strain properties, the said fibre and fabric samples were gamma-at a rate of 123.3 Krad•$h^{-1}$, the total dose being 2.97 Mrad. Before measuring, the samples were stored for 14 days. The tensile strength and elongation values, fabric weights, fibre gauge and draw ratio values measured before and after radiation are indicated in the Table 6. The stress-strain curves of the samples are given in the Figure 12. Because of the small number of the samples, the spread of the measuring values is considerable, but the results still show the general features characteristic for the method.

*I. a.* the following can be said of the radiation induced changes of the stress-strain values of the samples:

- The fabric elongations for the samples of the test series are very close and also largely independent of the corresponding fibre elongations.
- The radiation induced changes of the fabric elongations are very small and below the changes of the corresponding fibre elongation values.
- It is to be noted that the fibre structure of the samples 109A and 109D is already microfibrillar and thus outside the area of operation of the claimed method. Irrespective of this, the strength values of the fabric of the sample 109A are still satisfactory. The strength values of the fibre and fabric samples of the test series 109D, which lack the addition of stabilizing agent, have decreased considerably under the effect of radiation (the embrittlement of the fibre structure has already passed below the yield area values).
- The yield energy values (Iy, ws) of the fibre samples are before and after radiation respectively:

A4/211.9/156.2, D4/143.8/127.6, B1/46.0/45.5, B3/108.8/99.8, and F1/69.0/69.0. These values have been inserted in the fabric strength-yield energy function of Figure 5, which they also match satisfactorily.

Subexample 1.6

In the subexample 1.6, the effect of the preradiation temperature history of the fibre samples on the decrease of the mechanical values of the polymer due to gamma-excited chain oxidation is shown. The effect of the radiation or storage temperature on the chain oxidation rate is not discussed in this connection. An increase of the temperature of the fibre polymer in connection with any partial process stage of the production of the fabric or the fibre, results in an increase in the rate of the radiation ageing process of the polymer. This ageing process of the oriented polymer structure is difficult to evaluate due to its polyfunctional nature and also unclear origin. In view of the technical applicability of the production process of the gamma radiation resistant fibre, it is important to be able to estimate, at least qualitatively, the permissible area of operation of the method with respect to the temperature.

It is difficult to estimate the temperature reached by the fibre cable based on the machine temperatures used in the partial process stages of the fibre production. The measuring difficulties are primarily due to the speed of the fibre cable, material thickness as well as the weak heat transfer properties of polypropylene. In connection with this study, the so-called structural temperature, which is measured from the first order small-angle X-ray scattering (SAXS) of the polymer matrix of the fibre sample, has been used as the temperature of the fibre sample. The method is based on the observation that the so-called "long identity period" (L*, Å) of the polymer is monotonous function of the temperature ($\vartheta$,°C) of the form

$$L=66.0 - [5600/(\vartheta-187)]+[(4290/(298-\vartheta))-15.6]\log t \qquad /10/$$

The significance of the time (t, min), which is a variable in the equation, is very small taking into account the shortness of duration of the partial process stages, and under standard conditions one can take the unit time as its value.

In order to elucidate the temperature-operational range of the method, the behaviour of the fibre series 109B and 109C was studied during irradiation and during subsequent storage as a function of the pretreatment temperature of the fibre samples.

The polymer quality, testing conditions and some measuring results of the test series 109B are given in the Tables 2, 3 and 4. In the production of the fibre samples of the test series B and C the machine temperatures of the 2-godet (Fig. 3:5) were appr. 25 °C and 115 °C, respectively; the other conditions were identical for the series.

The heat treatment causes several changes in the fibre structure, that is *i.a.*: the fibre elongation decreases over the whole draw ratio range, the chain orientation changes, the degree of crystallinity increases, the stacking errors decrease, the interior tensions are relaxed, the number of interlamellar taut tie chains decreases, the lamellar structure is evened, and the SAXS-intensities increase. The changes occurring in the polymer structure and superstructure as a result of the temperature increasing have a significant effect on the radiation ageing, and the temperature effect under consideration is thus very complicated to its nature. For this reason a temperature function within the optimal

draw ratio-orientation-area of the new method was looked for, which would combine, into a single coefficient, the temperature dependency of the various partial processes affecting the fibre elongation.

A temperature function applicable to the samples of the test series 109B and 109C have the form (E, kJ; T, °K)

$$F'(T) = 1\text{-}9.659 \times 10^{5} \times \exp(-45.59/RT) \qquad /11/$$

The sample elongation corresponding to the treatment temperature of a partial process of the fibre production is, after radiation and storage,

$$\varepsilon_s(T) = [F'(T)/F'(T_o)] \times \varepsilon_s(T_o) = C_i \varepsilon_s(T_o) \qquad /12/$$

wherein $\varepsilon_s(T)$ and $\varepsilon_s(T_o)$ are the elongations corresponding to an increased and normal partial process temperature, respectively, after radiation and storage, F'(T) and F'($T_o$) are the values of the corresponding temperature functions and $C_i$ is the temperature coefficient.

The elongation of the fibre samples of the test series 109B is after irradiation (3.19 Mrad) and storage (Table 4: $\varepsilon_s$) of the form

$$\varepsilon_s(T_o) = 97.0 + (907.2 - 1193.7 \times fav)\, t^{-0.0998} \qquad /13/$$

The measured drawing temperature ($T_o$) for the test series 109B was, $\vartheta$ = 45°C, and the structural temperatures (T) of the heat treated fibre samples (2-godet) of the series were freely, $\vartheta$ = 91.0 °C and 119.0 °C. The structural temperature of the fibre samples of test series 109C corresponding to the higher machine temperature of the 2-godets (Fig.3:5) was $\vartheta$ = 111.6°C. Using the functions /11/ and /12/ one obtains (compared to the test series 109B: F'(318°K) = 0.9682) the following values for the temperature coefficients (T,°K/$C_i$) corresponding to the structural temperatures: 364/0.7436, 392/0.1857 and 384.6/0.3854).

The following table includes the calculated (equation /13/) post-radiation elongation values of the fibre samples using the orientation values of the test series 109B corresponding to the storage times of 24 days and 27 days. Using the said temperature coefficients and based on the elongation results of the 109B series, the elongation values of the heat-treated samples of the 109B series and of the 109C series produced on a hot-godet were calculated after corresponding radiation and storage. The table includes also the measured elongation values.

| Test Series | | fav | | |
|---|---|---|---|---|
| | Elongation result | 0.614 | 0.652 | 0.712 |
| 109B series | | | | |
| $\varepsilon_s$ | (318°K, 24 days) | 223.9 | 190.0 | 138.7 |
| $\varepsilon_s$ | (measured:318°K, 20 days) | 214.5 | 195.2 | 122.2 |
| $\varepsilon_s$ | (364°K, 24 days) | 166.5 | 142.0 | 103.1 |
| $\varepsilon_s$ | (measured: 364°K, 27 days) | 162.5 | 143.2 | 98.3 |
| $\varepsilon_s$ | (392°K, 24 days) | 41.6 | 35.4 | 25.8 |
| $\varepsilon_s$ | (measured: 392°K, 27 days) | 53.8 | 40.5 | 26.0 |
| 109C series | | | | |
| $\varepsilon_s$ | (384.8°K, 24 days) | 86.3 | 73.6 | 53.5 |
| $\varepsilon_s$ | (measured:384.8°K,24 days) | 89.8 | 64.6 | 51.5 |

According to the table, the values obtained with the applied temperature function and those measured correspond well to each other. In this connection the temperature dependency has been studied with respect to the drawing process, but exactly the same functionality can be observed with respect to the crimping, drying and fixation processes. It is to be observed that the highest fibre structural temperatures determines the degree of radiation ageing irrespective

of the partial process. For all the studied test series, a result analogous to that of the test series 109 has been obtained with respect to the partial process stages following the melt spinning.

The radiation ageing of the spinning fibre samples (1-godet) is to a fairly low degree dependant on the temperatures applied thereto during the preradiation heat treatment. This temperature dependency is to its origin obviously also different from the dependency discussed above. The magnitude of the SAXS-identity period and the corresponding peak intensity are mutually independent in the melt spinning process and the subsequent partial process stages, and thus the structural temperatures obtained therefrom differ from each other.

As regards the maximum preradiation structural temperature, and irradiation and the subsequent storage time, the fibre samples of the test series 109B follow the function /14/.

$$\varepsilon_s = -647.0 \text{ fav} + 33.18 \text{ x } t^{-0.0086} \text{ x } \exp(9.08/RT) \qquad /14/$$

The function is qualitative to its nature.

In this connection it is not purposeful to evaluate the reasons for the effect on the radiation ageing of the production temperatures in the partial process stages in the fibre production. It can, however, be observed that the heat treatment, besides raising the degree of crystallinity in the sample, strongly reduces the number of interlamellar taut tie chains. This reduction in the number of tie molecules corresponds to its thermal activation substantially the said temperature dependency of the 2-godet samples and on the other hand affects obviously significantly the radiation ageing. It is also to be noted that in the samples of the drawing process having a very low chain orientation, this temperature dependency can decrease significantly and correspondingly increase in spinning fibres with a high degree of orientation. This radiation ageing of the transitional area having a low thermal activation energy is partly combinable also with the degree of spherulite deformation and thus with the superstructure of the polymer matrix.

The changes in the tensile strength of the product fibres of the drawing process as a function of irradiation and subsequent storage time as well as the temperature of the preradiation heat treatment is studied. For the fibre samples of the test series 109B, a time and temperature function is obtained having the form ($\sigma_s$, mN/dtex) :

$$\sigma_s = 87.22 \text{ fav} - 38.61 \text{ x } t^{0.01234} \text{ x } \exp(-0.163/RT) \qquad /15$$

From the equation it can be seen that the effect of the pretreatment temperature on the tensile strength is very small (almost included within the scattering of the result). The small effect of the storage time on the tensile strength was expected. Although the compatibility of the product equation with the measured values is very good (t = 20-683 days, $\vartheta$ = 45 - 120°C, the result can be considered only qualitative due to the low number of samples.

Subexample 1.7

In the subexample 1.7 is shown that the radiation damage progresses logically as a function of the post-radiation storage time, the product being structurally stable at the different times of measurement and the measuring results being reproducible.

In the test series 43B according to the example, polypropylene polymer II was processed wherein the polymer VII was used as a plasticizing agent. The polymer characteristics are given in the Table 3. The polymer samples were stabilized in order to reduce radiation damage in the following manner: no 1/no additional stabilization, no. 4/0.25 % by weight Tinuvin 770 and 0.25 % by weight benzophenon, no. 6/0.25 % by weight Tinuvin 622 and 0.25 % by weight of benzophenon and no. 8/0.10 % by weight of Tinuvin 770 and 0.1 % by weight of benzophenon. The polymer II had been prestabilized with an antioxidation agent mixture: 0.13 % by weight Irgafos 168, 0.065 % by weight Irganox 1425 and 0.045 % by weight of Ca-stearate. The structural formulas of the stabilizing agents are given in the Table 1. The production conditions of the sample series no 43B are gven in the Table 3.

The samples were gamma-irradiated using a dose of 2.9 Mrad /121 Krad/h) and their strength values were measured during the post-radiation storage time. The used storage time series was (t, days): 9-41-75-189-365-420-930-1475.

The results for the tensile strength ($\sigma$, mN/dtex) and elongation ($\varepsilon$, %) of the radiated fibre samples can advantageously be expressed as a function of the storage time (t, days) in the form:

$$\sigma \text{ (or } \varepsilon) = -a_i \ln t + b_i \qquad /16$$

wherein $a_i$ and $b_i$ are coefficients.

The coefficients of the equation corresponding to the yield and tensile breaking strengths and elongations of the samples are indicated in the Table 7. The values for the tensile strength and elongation of the fibre samples before radiation (t = 0) and corresponding to storage time t = 1475 days, are indicated in the Table 7. For comparison reasons, the strength values of the samples corresponding to storage times of t = 1475 and t = 1825 days (5 years) calculated from the equation /16/ have been given in the table. According to the equations, the tensile strength values corresponding to both the yield and breaking limit decrease very little as function of storage time. The decrease of elongation at break as a function of time is fairly considerable and the angular coefficients of the corresponding equations are to their absolute values about a decade bigger than the coefficients of elongation at yield. The deviations of the values from each other are functions of both the draw ratio and the stabilizing agent additions.

The decrease of the tensile strengths and the elongations at the yield and breaking limits of samples corresponding to a storage time of five years, compared to the starting values are the following:

no / $-\Delta(\varepsilon)$, % ($-\Delta(\sigma)$, %):

yield limit: 1/29,5(12.7) - 4/15.0(7.5) - 6/11.9(21.3) - 8/19.9(9.5)

breaking limit: 1/55.7(42.7) - 4/30.8(34.7) - 6/26.8(35.4) - 8/48.9(39.8)

The changes of elongation at break of the samples during the maximum storage time are quite considerable. This is partly due to the high machine temperatures used in the production process for the test series being studied. It is to be observed that the tensile strength values of the fibre samples at the yield limit, which values are important in the production of water needled fabrics, decrease only to a low degree.

Example 2.

In the Example 2. is shown the effect of different composition and structural properties of the polypropylene polymer on the gamma-induced radiation oxidation.

First the test series no. 178 and 179 are studied. In the test series, long chained propylene polymers V and VI have been used, which differ from each other *i.a.* to their molecular weight distribution, the weight-average molecular weights being in each series the same. The polymer properties are indicated in the Table 2. The test run parameters for the test series are indicated in the Table 3 and the central result equations in the Table 4. In both test series, stabilizing agents were used so that the addition of each additive component was 0.15 % by weight. With respect to the stabilizing agents, the test series were marked with the letters A-D, the corresponding stabilizing mixtures being A: no additives, B: Chimassorb 944 + Tinuvin 622, C: Chimassorb 944 + Tinuvin 770 and D: Chimassorb 81 + Tinuvin 770. In the last mentioned series, the additive concentrations were: $D_1$: 0.075 % by weight and $D_2$: 0.150 % by weight. The structural formulas of the stabilizing agents are given in the Table 1.

In the subexample 1.3 is shown how the stress-strain relationships change as a function of radiation and subsequent storage time. According to the example, both the stress and the strain values on radiation change above the yield limit following the stress-strain relationships of the non-radiated starting fibre. Hereby the magnitude of radiation damage directed to the tensile strength of the fibre sample is much lower than that directed to the elongation. It can be shown that the post-radiation tensile strength ($\sigma_s$, mN/dtex) with respect to the storage time (t, days) follows quite accurately the function:

$$\sigma_s/\sigma_o = K_t = c_i t^{-ni} \qquad /17/$$

The preradiation tensile strength ($\sigma_o$, mN/dtex) is a function of the average orientation factor of the form

$$\sigma_o = a_i fav + b_i \qquad /18/$$

The coefficients of the equations are indicated in the Table 4. The values for the coefficients corresponding to the reference time, t = 30 days, and radiation dose 3.16 Mrad, referring to both test series, are also indicated in the table. The radiation damage on the tensile strength ($(\sigma_o-\sigma_s)/\sigma_o = -\Delta(\sigma)$, %) is according to the equation /17/ of the form

$$-\Delta(\sigma) = 100(1-c_i t^{-ni}) \qquad /19/$$

The Table 4 shows the elongation values measured before and after in the said radiation conditions for both test series in accordance with equation /7/. When modifying the storage times for test series no 178 to the reference

time, a form corresponding to equation /8/ has been used:

$$\varepsilon_s = b_i + (0.76 - fav) \times c_i \times t^{-ni} \qquad /20/$$

wherein the coefficients for the test series 178B, 178C and $178D_2$ are respectively: $b_i$/136, 119 and 133, $c_i$/933.1, 795.8 and 944.6, $n_i$/0.100.

In the Figure 13, the radiation damage to the fibre elongation ($-\Delta(\varepsilon)$, %), for both test series corresponding to a radiation dose of D = 3.16 Mrad and a storage time, t = 30 days, are marked. In the figure also the fibre cable tension from spinning is given as a function of the average orientation of the fibre. From the Fig. 13 it can be seen that the radiation damage of the fibre samples from the test series no. 179, especially at high orientation values, is very high both in samples containing additional stabilization and in samples containing no additional stabilization. The fibre sample series of the test series 178 show less radiation damage than the samples of the series no. 179 in all conditions. According to the figure, a much higher cable tension is needed in the melt spinning process for the fibre samples of the test series no. 179 than for the test series no. 178 in order to obtain a fibre sample orientation of the same magnitude.

In the Figure 14, the radiation damage to the tensile strength of the fibre samples (in the said dose conditions) are given for both test series. In the same figure, also the changes in the X-ray crystallinity degree of the samples of the test series, as a function of the average orientation, are given. According to Fig. 14, the degree of crystallinity of the fibre samples of the test series 179 is, over the whole studied range, substantially higher than the degree of crystallinity of the test series no. 178. The degree of crystallinity of the spinning fibres of the test serie no. 179 is almost constant as a function of average orientation. The degree of crystallinity ($\chi$, %) generated during the spinning of the fibre samples of test series no. 178, is an increasing function of spinning tension ($\sigma_k$, $\mu$N/dtex), but for the fibre samples of the test series no. 179, the degree of crystallinity remains constant at increasing cable tension. The values of the degree of crystallinity of the fibre samples of the test series of the Example 2 are given in Table 8. From the results in the Fig. 14 it may be concluded that within accuracy of measurement the radiation damage directed on the tensile strength of the samples from both test series is substantially of similar magnitude (the radiation damage on the elongation, on the other hand, was of substantially different magnitude for the samples of the two test series).

The effect of various structural and processing factors on the $e_o$, $e_s$/fav-function of the equation /7/ is studied. The polymer characteristics, the test run conditions and some central results of the test series no 109B, C, D and F, no. 110 L and i and no. 127 are indicated in the Tables 1, 2, 3 and 4, respectively. In the test series, in addition to the basic stabilization for the polymer, the compounds Tinuvin 770 and technical grade benzophenon, were used as a stabilizing agent additive, both at an amount of 0.3 % by weight. In the test series 109 D, no additional stabilization was used.

The path of the functions $\varepsilon_{o,s} = a_i$ fav + $b_i$ before and after radiation and subsequent storage determines the magnitude of radiation damage on elongation.

When comparing the inclinations of the elongation functions ($\varepsilon_o$/fav) of the test series (Table 4.) it can be seen that

- the elongation values of both the 1-godet and the 2-godet samples of the series 178 fall on the same straight line. The inclination of the line increases due to the additions of stabilizing agent, whereby both the amount and type of additive has an effect. The control of cable tension affects in matching *i.a.* the 1- and 2-godet elongation values in the same $\varepsilon_o$/fav function. It is, however, then to be observed that in a spinning fibre formed through solidification from a melt, the contributing factors determining the magnitude of orientation are different from those produced during drawing of a solid product.
- the elongation function of the 2-godet fibre samples of the series no. 179 is substantially parallel to that of the samples of the no. 178 series. The angular coefficient of the function for the 1-godet samples is high, that is, compared to the angular coefficient of the function for the 2-godet samples, almost about 400 % bigger (l-al: 3280/1-god, 653/2-god). For the elongation functions of the test series no. 109 F and 110 L, the angular coefficients (within the range of accuracy of measurment) are of the same magnitude as those of the functions for the test series no. 179. In the test series 109 F and 110 L the same polymer (III) has been used (III), which to its MWD-values is quite close to the polymer of the series no. 179 (VI: Table 2). From Table 8 it can be seen that the samples of these test series have a degree of crystallinity which is substantially higher than that of others, and which remains constant both as a function of orientation and of the spinning draw ratio. It is to be observed that correspondingly both the station and intensity values of the first order SAXS scattering of the spinning fibre samples of the test series are on a higher level than others. By comparing the test conditions and results of the tables it can be seen that, in the range studied, changes in capacity, spinning draw ratio, fibre gauge, or amounts and quality of additives, have only a small effect on the elongation function. Thus the inclination of the elongation function of the spinning

samples before radiation is largely determined by the degree of crystallinity and the changes thereof.

- the mutually parallel elongation functions of the 2-godet samples of the test series no. 178 A, 179 A and 127 deviates in particular from the directions of the elongation functions of the series no. 109 and 110. The degree of crystallinity of the spinning samples of the test series no. 179 A and 109 F being higher than that of the others, and is present also in the draw fibre samples. Hereby the degree of crystallinity is, however, a function of orientation and decreases with the series no 109 F more steeply than with the series no. 179. For the samples of the test series 109 and 110 (with the exception of the series F and L), the degree of crystallinity is lower than the values of the afore mentioned test series, its change as a function of orientation being substantially of the same magnitude. The degree of crystallinity of the samples of the test series no. 178 corresponds primarily to the values of the samples of the series no. 109, but is, as a function of orientation, less steep. The molecular weight distribution of the polymer and the degree of crystallinity of the draw fibre and the changes thereof affect in the direction of the elongation function of the fibre samples. Besides these, a decisive effect on the angular coefficient of the elongation function is exerted by the amount and quality of the adjuvants and stabilizing agents.

Although the preradiation behaviour of the elongation function of the fibre samples is polyfunctional, it has in this connection been possible to qualitatively show the major behavioral characteristics. Much more difficult and ambiguous is the examination of the elongation function after radiation and storage. The chain orientation of the fibre samples does not substantially change on radiation. The crystallinity values of the samples may increase slightly. The radiation induced change of direction of the elongation function is thus apparently dominated by phenomena associated with radiation oxidation.

Under the influence of radiation, the angular coefficient of the elongation function of the samples can, compared to the initial value ($\varepsilon_o$/fav), remain of the same magnitude (*i.a.* 173 A), increase (*i.a.* 179 B, C and D, and generally with series when fav > 0.76), or decrease (*i.a.* 178 A, B, C and D series often when fav < 0.76 and most often with spinning fibres). Hereby changes in the same direction are seen also in the magnitude of the radiation damage.

The primary stabilization agent system and the additives (plasticizing and stabilizing agents) both to their quality and amount, in combination with the morphological factors of the samples, affect the position and direction of the elongation function of the fibre samples. Many of these factors, such as the solubility and diffusion coefficient values of oxygen and additives as a function of the orientation and superstructure of the polymer matrix, are almost unknown. Also the translational velocities of the chain segments and radicals in the polymer matrix are only qualitatively known. Of the factors affecting especially radical oxidation, *i.a.* the following may be mentioned:

- under the influence of the molecular chain orientation, both the solubility and the diffusion rates of oxygen and stabilizing agents decrease. It is thus to be observed that in the superstructure of the operational range of the method, *i.a.* material velocities and solubilities of the intra- and the interspherulitic amorphous areas differ from each other and, in addition, are functions of the degree of deformation.
- a decrease in the degree of crystallinity raises the amount of stabilizing agent dissolved in the amorphous phase and thus reduces the amount of discharge material following supersaturation (of used stabilizing agents, *e.g.* TV 770). The degree of crystallinity decreasing, also the importance of the taut tie molecules and simultaneously the degree of localized radiation damage, decreases.
- even though the chain orientation is high in the operational range of the method, the decrease in polymer matrix density, which follows the decrease in the degree of crystallinity, results in an increase in the free volume, simultaneously increasing the radical termination rate.

In the method according to the invention, the object is to decrease the degree of crystallinity of the spinning fibre by regulating the spinning cable tension and the cooling rate of the polymer melt (regulation of the amount and temperature of the cooling air). Hereby already in the spinning stage, supersaturation of the stabilizing agents, as well as their discharge formations is reduced. The slight additional decrease in the degree of crystallinity appearing in the drawing stage following the spinning stage, substantially affects the distribution of stabilizing agents in the amorphous matrix and thus the decrease of radiation damages.

The efficacy of the stabilizing agents used in the Example 2 can be evaluated i.a. from both the tensile strength and the elongation radiation damage values based on the coefficients of the equations /19/ and/or /20/ (Table 4). Based on the radiation damages for the test series 178 and 109 B, when using a phosphite-phosponite basic stabilization, the order of superiority of the added radiation stabilization agents is:

Tinuvin 770 + benzophenon - Tinuvin 770 + Chimassorb 81 (benzophenon) - Tinuvin 770 + Chimassorb 944 (both hindered amines) - Tinuvin 622 + Chimassorb 944.

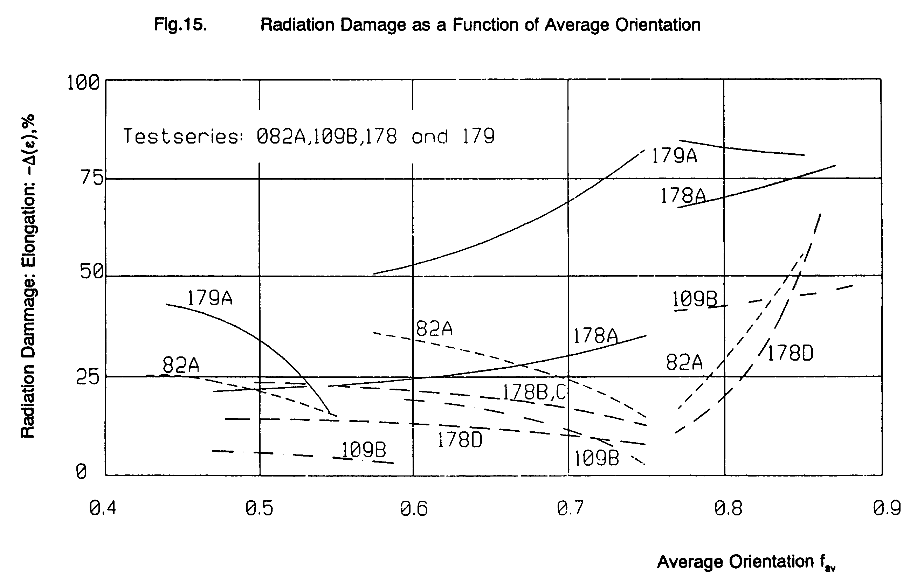

In the Figure 15, the magnitude of radiation damages of the samples of the test series no. 82 A and 109 B based on the results of the Table 4 have been given as a function of the chain orientation. The elongation functions of the 2-godet samples of the test series no. 109 B before and after radiation converge when the orientation increases

close to the transformation area: deformed spherulite-/microfibrillar structure, and simultaneously the radiation damage approaches zero. In the figure one can also see the generation of radiation damages in the test series no. 179 A, 178 A and D as a function of the chain orientation.

The effect of preradiation heat treatment of the fibre and/or the fabric on radiation damage will still be studied briefly. In the subexample 1.6, the effect of preradiation heat treatment on the post-radiation strength values of the samples of the test series no. 109 B and 109 C, has been studied.

The temperature behaviour of the samples of the test series no. 82 A and 82 C (Tables 2, 3, 4, 5 and Figure 15) where the temperature of the godets are 25°C and 121°C, respectively, corresponds well to that of the test series no. 109 B and 109 C. In the test series 82 C, the high temperature of the godets expects the post-radiation sample elongations to be almost elongations at the yield limit (and thus also high radiation damage values) already at low chain orientation values. Hereby the decrease of the radiation damage is considerable at increased chain orientation, especially over the whole 2-godet draw area. Within the limits of accuracy of measuring, this radiation damage is almost independent of the postradiation storage time (82 C: t = 28, 132, 195, 277, 607 days). For the samples of the test series 82 C, the radiation damage is a function of the average orientation factor: $-\Delta(\varepsilon)/100 = 1.6151-1.0547$ fav (*i.a.* t = 195: Table 5). When heat treating the samples of the test series no. 82 C at high temperatures, the radiation damage decreases even further: for example the conditions $\vartheta = 140°C/t = 30^m$: $-\Delta(\varepsilon)/100 = 2.4468-2.2596$ fav. When heat treating the samples of the test series no. 82 A at corresponding temperatures, the same phenomena can be observed - except at the lowest chain orientations - as with the samples of the test series no. 82 C.

The observed temperature effect is at least partly due to *i.a.* the termination of work-generated radicals due to the heat treatment. It is self-evident that in the microfibrillar area the heat treatment effect can be utilized in the production of gamma-sterilizable fibres and fabrics. As the elongation values of the samples both after heat treatment and radiation are already fairly low and close to the yield limit, especially in the microfibrillar superstructure area, the already rather small possibilities in the said area for regulating both fibre and fabric elongations and thermal contractions are lost. Also the cost factors have to be considered if the heat treatment is carried out as a separate lasting annealing heating. The said heat treatment method can be used for the production of very strong fibres and fabrics having low elongations, for special purposes.

In the state of the art it has been concluded that in specific cases also gamma-sterilizable, thermally bonded fabrics can be produced from polypropylene fibres. Hereby, however, fibres drawn to as low a orientation degree as possible, or spinning fibres have to be used, in order to partially eliminate the effect of the temperature of the thermal bonding process on the tie points and the fibres. If necessary, a heat treatment has to be applied to the thermo-bonded non-woven fabric before radiation, whereby *i.a.* the radicals generated through strong shear in working during the bonding are terminated. The fibres to be thermo-bonded have to be radiation stabilized in addition to their basic stabilization.

In the Examples 1 and 2 the carrying out of the method has been disclosed as regards both the fibre and the fabric. It is to be observed the very complicated process according to the method can be varied in very many ways while still remaining within the operational area delimited by the disclosed examples and the patent claims.

In connection with the examples there has been an attempt to theoretize phenomena associated with the method, in order to provide a clear natural-science picture of the method according to the invention. It is, however, self-evident that there has been no attempt to explain all phenomena of the method, either due to their unknown nature or to the lack of sufficient technical-scientific measuring results, wherefore it is not possible to rely only on the argumentation given in the description of the method.

Table 1. Structure of stabilizing agent

| Structure of compound | Molecular weight | Melting range °C | Formula and commercial name of compound |
|---|---|---|---|
| 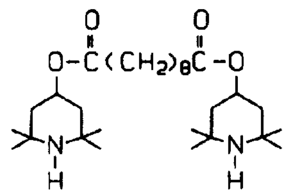 | 481 | 81 - 85 | Sis [2,2,6,6-tetramethyl-4-piperidyl] sebacate<br><br>Tinuvin 770 |
| 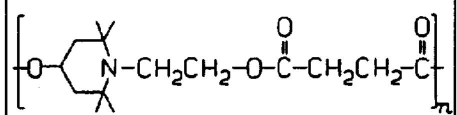 | appr.3500 | 55 - 70 | Butanedioic acid, polym.w. 4-hydroxy-2,2,6,6-tetramethyl-1-piperidineethanol<br>Tinuvin 622 LD |
| 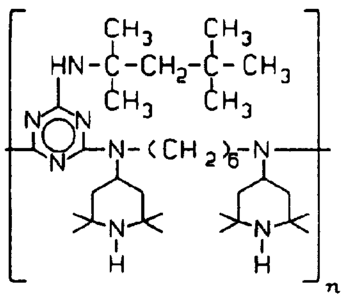 | appr.3000 | 100 - 135 | Poly-[ [6-[(1,1,3,3-tetramethylbutyl)-imino]-1-3-5-triazin-2,4-diyl] [2-(2,2,6,6-tetramethylpiperidyl)-imino]- hexamethylen-[4-(2,2,6,6-tetramethylpiperidyl)-imino]]<br><br>Chimassorb 944 LD |

Table 1 - continued

| Structure of compound | Molecular weight | Melting range °C | Formula and commercial name of compound |
|---|---|---|---|
| 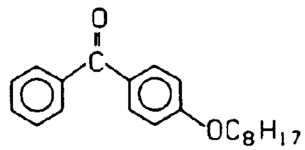 | 326.4 | 48 | 2-hydroxy-4-n-octyloxybenzophenone<br>Chimassorb 81 |
| 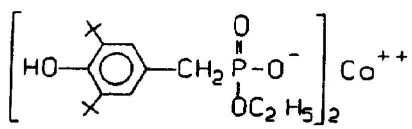 | 69.5 | >260 | Phosphonic acid, [3,5-bis(1,1-dimethylethyl)-4-hydroxyphenyl]-,monoethyl ester<br>Irganox 1425 |
| 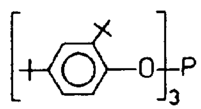 | 647 | 180-186 | Phenol,2,4-bis(1,1-dimethylethyl)-,phosphite(3:1)<br>Irgafos 168 |
| 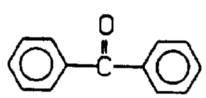 | 182.3 | 48 | Benzophenone |

Table 2. Molecular Weight of Test Polymers

| Polymer Test Series | | | Melt Index 1) | Molecular Weight 2) | | |
|---|---|---|---|---|---|---|
| | | | | $M_w$ | $M_n$ | $M_w/M_n$ |
| I | 82 | 057A | 25 | 207700 | 35320 | 5.88 |
| II | 82A, 109B | 057B | 19 | 192700 | 48100 | 4.01 |
| III | | 109F | 18 | 249000 | 49100 | 5.07 |
| IV | | 127 | 18 | 216000 | 71000 | 3.04 |
| V | | 178 | 18 | 223500 | 70800 | 3.16 |
| VI | | 179 | 18 | 221000 | 48200 | 4.59 |
| VII | | - | 420 | 98400 | 22150 | 4.44 |

1) MFR: ASTM D1238, 230°C/21.6 N; g/10 min

2) $M_w$ and $M_n$: weight and number average molecular weight

$M_w/M_n$ = D: molecular weight distribution

Table 3. Test Run Conditions

| Test Run Condition | | Figure 3 Station | 057 | 82A | Test Series 82 C | 109B | 109F |
|---|---|---|---|---|---|---|---|
| Polymer Pump | : $n_r$, $min^{-1}$ | | 15.18 | 6.30/15.15 | 6.30/15.15 | 11.69 | 11.69 |
| Nozzle Speed | : $v_s$, $m \cdot min^{-1}$ | | 0.3812 | - | - | 0.2936 | 0.2936 |
| 2-Godet Speed | : $v_2$, $m \cdot min^{-1}$ | 5 | 88.0 | - | - | 90.2 | 90.0 |
| 1-Godet Speed | : $v_1$, $m \cdot min^{-1}$ | 3 | - | 30.2 | 30.3 | - | - |
| Draw ratio | : Drawing/spinning | | | | | | |
| Series no | 1 | | 1.30/178 | 1.24/189 | 1.24/189 | 1.25/247 | 1.24/247 |
| | 2 | | 2.00/116 | 1.50/157 | 1.50/157 | 1.51/204 | 1.50/204 |
| | 3 | | 2.50/ 92 | 2.00/118 | 2.00/119 | 2.01/153 | 2.00/153 |
| | 4 | | 3.00/ 77 | 2.50/ 94 | 2.50/ 94 | 2.49/123 | 2.50/123 |
| | 5 | | - | 3.00/ 79 | 3.00/ 79 | 2.91/105 | 2.99/103 |
| | 6 | | - | | | 2.98/103 | |
| Temperature | : $\vartheta$, °C | | | | | | |
| - 2-Godet | | 5 | "25" | "25" | 121 | "25" | "25" |
| - Drawing Oven | | 4 | 94 | 80 | 80 | 56 | 58 |
| - Drying Oven | | 8 | 95 | 69 | 83 | 55 | 51 |

Table 3. (Continued)

| Test Run Condition | | Figure 3 Station | 109C | 110i | Test Series 127C | 178 | 179 |
|---|---|---|---|---|---|---|---|
| Polymer Pump | : $n_r$, $min^{-1}$ | | 11.69 | 15.15 | 15.15 | 11.03 | 15.15 |
| Nozzle Speed | : $v_s$, $m \cdot min^{-1}$ | | 0.2936 | 0.3812 | 0.3812 | 0.2769 | 0.3804 |
| 2-Godet Speed | : $v_2$, $m \cdot min^{-1}$ | 5 | 90.0 | 89.9 | 116.5 | 65.4 | 89.7 |
| 1-Godet Speed | : $v_1$, $m \cdot min^{-1}$ | 3 | - | - | | | |
| Draw Ratio | : Drawing/Spinning | | | | | | |
| Series no. | 1 | | 1.24/247 | 1.20/197 | 1.20/258 | 1.25/189 | 1.15/205 |
| | 2 | | 1.49/206 | 1.50/158 | 2.00/154 | 1.50/157 | 1.25/189 |
| | 3 | | 2.00/153 | 2.00/118 | 2.50/123 | 2.00/118 | 1.50/158 |
| | 4 | | 2.44/125 | 2.49/ 95 | 3.00/102 | 1.50/ 95 | 2.00/118 |
| | 5 | | 2.96/103 | 3.02/ 78 | - | 3.00/ 79 | 2.50/ 96 |
| | 6 | | - | - | - | - | 3.00/ 79 |
| Temperature | : °C | | | | | | |
| - 2-Godet | | 5 | 114 | "25" | "25" | "25" | "25" |
| - Drawing Oven | | 4 | 56 | 57 | (60) | (60) | (60) |
| - Drying Oven | | 8 | 69 | 55 | (50) | (50) | (50) |

Table 4.1)

$\varepsilon_o$ ($\varepsilon_s$, $\sigma_o$, $\sigma_s$) = $a_i$ fav + $b_i$+ $c_i$ $t^{-ni}$ = F(1)

$\varepsilon_o$ ($\varepsilon_s$, $\sigma_o$, $\sigma_s$) = $b_i$+ (0.76-fav)$c_i$ $t^{-ni}$ = F(2)

| Example | Function | Coefficients | | | |
|---|---|---|---|---|---|
| Station, Limits | | -a | b | c | |
| No 82 A | | | | | |
| 1-Godet: | | | | | |
| t = 0 | $\varepsilon_o$ | 1909.4 | 1285.5 | | |
| t = 271 | $\varepsilon_s$ | 1210.3 | 860.3 | | |
| t = 14-805 | $F_\varepsilon(1)$ | 1210.3 | - | 871.6 | 0.0023 |
| 2-Godet: | | | | | |
| fav < 0.76 | $\varepsilon_o$ | 737.5 | 692.5 | | |
| fav > 0.76 | $\varepsilon_o$ | 345.5 | 394.6 | | |
| t = 194 | | | | | |
| fav < 0.76 | $\varepsilon_s$ | 187.5 | 257.5 | | |
| fav > 0.76 | $\varepsilon_s$ | 774.6 | 703.7 | | |
| fav < 0.76 | $F_\varepsilon(2)$ | | 115 | 737.6 | 0.2600 |
| fav > 0.76 | $F_\varepsilon(2)$ | | 115 | 840.9 | 0.0220 |
| No 109 B | | | | | |
| 1-2 Godet: | | | | | |
| ($K_{30}$) | $\sigma_s/\sigma_0$ | (0.7966) | | 0.8699 | 0.0259 |
| 1-Godet: | | | | | |
| | $\sigma_o$ | 3.3 | 19.65 | | |
| | $\varepsilon_o$ | 775.9 | 745.3 | | |
| t = 10-679 | $F_\varepsilon(1)$ | 647.0 | | 677.6 | 0.0086 |
| 2-Godet: | | | | | |
| t = 0 | $\sigma_o$ | -125.7 | -58.7 | | |
| fav < 0.76 | $\varepsilon_o$ | 1193.8 | 1004.6 | | |
| fav > 0.76 | $\varepsilon_o$ | 300.0 | 325.0 | | |
| fav < 0.76 | $F_\varepsilon(2)$ | | 97.0 | 1193.7 | 0.0998 |
| fav > 0.76 | $\varepsilon_s$ | 208.3 | 215.3 | | 0 |
| Station, Limits | | -a | b | c | n |
| No 109 F | | | | | |
| 1-2 Godet: | | | | | |
| t = 26-744 | $\sigma_s/\sigma_o$ | (0.8229) | | 0.9171 | 0.0318 |
| 1-Godet: | | | | | |
| t = 0 | $\sigma_o$ | -4.29 | 16.48 | | |
| 2-Godet: | | | | | |
| t = 0 | $\sigma_o$ | -72.22 | -26.33 | | |
| No 127 | | | | | |
| 1-2 Godet: | | | | | |
| t = 19-471 | $\sigma_s/\sigma_o$ | | | 0.8656 | 0.0230 |
| 1-Godet: | | | | | |
| t = 0 | $\sigma_o$ | -40.00 | -2.74 | | |
| 2-Godet: | | | | | |
| t = 0 | $\sigma_o$ | -120.48 | -55.89 | | |
| No 178 | | | | | |

Table 4.1) (continued)

| Station, Limits | | -a | b | c | n |
|---|---|---|---|---|---|
| 1-2 Godet: | | | | | |
| A ($K_{30}$) | $\sigma_s/\sigma_0$ | (0.6195) | | | |
| B | $\sigma_s/\sigma_0$ | (0.6841) | | 0.7623 | 0.0318 |
| C | $\sigma_s/\sigma_0$ | (0.6923) | | 0.7715 | 0.0318 |
| D1 | $\sigma_s/\sigma_0$ | (0.6981) | | 0.7290 | 0.0127 |
| D2 | $\sigma_s/\sigma_0$ | (0.7650) | | 0.7989 | 0.0127 |
| 1-Godet: | | | | | |
| A | $\sigma_o$ | -22.73 | 8.50 | | |
| B | $\sigma_o$ | -26.70 | 3.51 | | |
| C, D | $\sigma_o$ | -25.38 | 5.02 | | |
| Station, Limits | | -a | b | | |
| 2-Godet: | | | | | |
| A | $\sigma_o$ | -117.61 | -53.07 | | |
| B | $\sigma_o$ | -117.61 | -54.44 | | |
| C | $\sigma_o$ | -117.61 | -54.08 | | |
| D | $\sigma_o$ | -117.61 | -56.12 | | |
| | | | | | |
| A | $\varepsilon_o$ | 702.9 | 691.5 | | |
| fav < 0.76 | $\varepsilon_s$ | 640.0 | 586.4 | | |
| fav > 0.76 | $\varepsilon_s$ | 340.0 | 310.4 | | |
| B | $\varepsilon_o$ | 976.1 | 896.8 | | |
| fav < 0.76 | $\varepsilon_s$ | 734.6 | 694.3 | | |
| fav > 0.76 | $\varepsilon_s$ | 1683.3 | 1415.3 | | |
| C | $\varepsilon_o$ | 830.0 | 767.0 | | |
| fav < 0.76 | $\varepsilon_s$ | 603.9 | 577.9 | | |
| fav > 0.76 | $\varepsilon_s$ | 1850.0 | 1525.0 | | |
| D | $\varepsilon_o$ | 830.0 | 775.9 | | |
| fav < 0.76 | $\varepsilon_s$ | 707.7 | 670.9 | | |
| fav > 0.76 | $\varepsilon_s$ | 1083.3 | 956.3 | | |
| | | | | | |
| No 179 | | | | | |
| 1-2 Godet: | | | | | |
| A ($K_{30}$) | $\sigma_s/\sigma_o$ | (0.5923) | | | |
| B | | (0.6800) | | | |
| C | | (0.6650) | | | |
| D | | (0.7075) | | | |
| | | | | | |
| 1-Godet: | | | | | |
| A-D | $\sigma_o$ | -15.91 | 6.17 | | |
| 2-Godet: | | | | | |
| A-D | $\sigma_o$ | -72.23 | -24.79 | | |
| t = 30 | | | | | |
| | | | | | |
| 1-Godet: | | | | | |
| A | $\varepsilon_o$ | 3280.0 | 2041.8 | | |
| | $\varepsilon_s$ | 1240.0 | 887.4 | | |
| B | $\varepsilon_o$ | 16380.0 | 8987.2 | | |
| | $\varepsilon_s$ | 4100.0 | 2424.1 | | |
| C | $\varepsilon_o$ | 2259.4 | 1621.0 | | |

Table 4.1) (continued)

| Station, Limits | | -a | b | | |
|---|---|---|---|---|---|
| | $\varepsilon_s$ | 395.3 | 501.7 | | |
| D | $\varepsilon_o$ | 9550.0 | 5290.5 | | |
| | $\varepsilon_s$ | 12180.0 | 6585.0 | | |
| 2-Godet: | | | | | |
| A | $\varepsilon_o$ | 653.3 | 673.7 | | |
| | $\varepsilon_s$ | 661.5 | 527.8 | | |
| B | $\varepsilon_o$ | 1322.2 | 1091.3 | | |
| | $\varepsilon_s$ | 1182.2 | 873.6 | | |
| C | $\varepsilon_o$ | 603.4 | 631.9 | | |
| | $\varepsilon_s$ | 1244.0 | 937.1 | | |
| D | $\varepsilon_o$ | 1015.4 | 855.9 | | |
| | $\varepsilon_s$ | 1238.5 | 924.8 | | |
| 1-Godet: | | | | | |
| 109 B | $\varepsilon_o$ | 775.9 | 745.3 | | |
| 109 F | $\varepsilon_o$ | 3280.0 | 2095.0 | | |
| 110 L | $\varepsilon_o$ | 3280.0 | 2042.0 | | |
| 127 | $\varepsilon_o$ | 702.9 | 681.5 | | |
| 178 A | $\varepsilon_o$ | 702.9 | 691.5 | | |
| 179 A | $\varepsilon_o$ | 3280.0 | 2041.8 | | |
| 2-Godet: | | | | | |
| 109 B | $\varepsilon_o$ | 1193.8 | 1004.6 | | |
| 109 C | $\varepsilon_o$ | 1111.1 | 964.4 | | |
| 109 D | $\varepsilon_o$ | 1100.0 | 977.0 | | |
| 109 F | $\varepsilon_o$ | 1093.3 | 925.9 | | |
| 110 L | $\varepsilon_o$ | 1020.0 | 915.0 | | |
| 110 I | $\varepsilon_o$ | 1195.0 | 1038.9 | | |
| 127 | $\varepsilon_o$ | 702.9 | 681.5 | | |
| 178 A | $\varepsilon_o$ | 702.9 | 681.5 | | |
| 179 A | $\varepsilon_o$ | 653.3 | 673.7 | | |
| 1) In this table, fibres having fav > 0.76 are comparative fibres | | | | | |

Table 5.2)

| Measurement | Draw Sample | | | | |
|---|---|---|---|---|---|
| Point | 1 | 2 | 3 | 4 | 5 |
| No 82 A: 1-Godet | | | | | |
| $\lambda_k$ | 188.9 | 157.0 | 118.2 | 93.6 | 78.6 |
| fav | 0.543 | 0.516 | 0.475 | 0.441 | 0.416 |
| $\varepsilon_o$, % | 254.4 | 328.2 | 379.9 | 368.1 | 477.8 |
| $\varepsilon_s$ (271 Days), % | 210.6 | 246.7 | 298.3 | 333.9 | 342.0 |
| No 82 A: 2-Godet | | | | | |
| $\lambda$ | 1.25 | 1.50 | 2.00 | 2.50 | 3.00 |
| fav | 0.598 | 0.636 | 0.715 | 0.805 | 0.870 |
| $\varepsilon_o$, % | 252.0 | 221.5 | 165.3 | 116.5 | 94.1 |
| $\varepsilon_s$ (194 Days), % | 145.6 | (137.0) | 123.9 | 78.8 | 29.9 |

Table 5.2) (continued)

| Measurement | Draw Sample | | | | |
|---|---|---|---|---|---|
| No 82 C: 2-Godet | | | | | |
| $\lambda$ | 1.25 | 1.50 | 2.00 | 2.50 | 3.00 |
| fav | 0.702 | 0.730 | 0.787 | 0.843 | 0.900 |
| $\varepsilon_o$ | 177.6 | 149.4 | 106.9 | 94.4 | 75.8 |
| $\varepsilon_s$ (195 Days), % | 21.7 | 23.4 | 24.1 | 25.6 | 24.5 |
| 2) In this table, fibres having fav > 0.76 are comparative fibres. | | | | | |

Table 6. Tensile Strength - Elongation Values of Fabrics and Fibres before and after Irradiation.

| Sample | Treatment | w, g•m$^{-2}$ | Fabric σ∥ /σ⊥, N | ε∥ /ε⊥, % | λ | Fibre d,dtex | σ,mN/dtex | ε, % |
|---|---|---|---|---|---|---|---|---|
| F-1: | Initial | 46.9 | 69.0/31.3 | 103.9/173.4 | 1.24 | 1.90 | 15,6 | 295.4 |
| | Irradiation | 46.8 | 68.9/26.7 | 92.1/144.1 | | 2.02 | 12.0 | 259.1 |
| | -Δ, % | | 0.1/14.7 | 11.4/ 16.9 | | | 23.1 | 12.3 |
| B-1: | Initial | 45.3 | 87.4/28.9 | 150.1/174.5 | 1.25 | 1.79 | 18.5 | 247.4 |
| | Irradiation | 45.9 | 70.4/26.5 | 103.0/146.4 | | 1.84 | 14.6 | 220.3 |
| | -Δ, % | | 19.5/ 8.3 | 31.4/ 16.1 | | | 21.1 | 11.0 |
| B-3: | Initial | 45.0 | 110.0/30.3 | 107.4/151.8 | 2.00 | 2.04 | 27.2 | 163.2 |
| | Irradiation | 45.6 | 82.1/29.9 | 80.8/119.6 | | 1.82 | 20.9 | 111.1 |
| | -Δ, % | | 25.4/ 1.3 | 24.8/ 21.2 | | | 23.2 | 31.9 |
| D-4: | Initial | 44.3 | 91.0/32.8 | 99.9/151.4 | 2.50 | 1.90 | 32.2 | 108.6 |
| | Irradiation | 42.3 | 58.9/25.0 | 68.3/116.0 | | 1.95 | 22.6 | 52.4 |
| | -Δ, % | | 35.3/23.8 | 31.6/ 23.4 | | | 30.0 | 51.8 |
| A-4: | Initial | 45.0 | 100.8/27.4 | 106.6/154.0 | 2.50 | 2.11 | 37.7 | 110.3 |
| | Irradiation | 46.0 | 91.8/29.0 | 88.3/125.7 | | 2.25 | 21.7 | 64.8 |
| | -Δ, % | | 8.9/-5.8 | 17.2/ 18.4 | | | 42.4 | 38.0 |

Table 7. Storage Ageing

| Fibre Sample | Tensile Strength (σ) $a_i$ | Tensile Strength (σ) $b_i$ | Elongation (ϵ) $a_i$ | Elongation (ϵ) $b_i$ | Symbol | Storage Time (t) 0 | 1475 | (1475) | (1825) |
|---|---|---|---|---|---|---|---|---|---|
| **Yield Limit nr.** | | | | | | | | | |
| 1 | | | 0.9452 | 31.30 | σ | 11.30 | 9.88 | 9.41 | 9.86 |
| | | | | | ϵ | 34.30 | 28.0 | 24.26 | 24.20 |
| 4 | 0.1807 | 10.73 | 0.4339 | 29.52 | σ | 10.80 | 10.05 | | 9.99 |
| | | | | | ϵ | 30.90 | 26.0 | 26.35 | 26.26 |
| 6 | | | 0.3532 | 30.58 | σ | 11.40 | 10.29 | | 8.97 |
| | | | | | ϵ | 31.70 | 28.0 | 28.00 | 27.93 |
| 8 | 0.1671 | 18.12 | 0.6492 | 32.74 | σ | 18.65 | 16.89 | 16.90 | 16.87 |
| | | | | | ϵ | 34,80 | 28,0 | 28.00 | 27.86 |
| **Breaking Limit: nr.** | | | | | | | | | |
| 1 | 0.7494 | 16.37 | 12.264 | 196.2 | σ | 18.75 | 10.9 | 10.9 | 10.74 |
| | | | | | ϵ | 235.2 | 101.9 | 106.7 | 104.1 |
| 4 | 0.6349 | 17.53 | 7.4527 | 235.2 | σ | 19.55 | 12.9 | 12.9 | 12.76 |
| | | | | | ϵ | 258.9 | 173.3 | 180.8 | 179.2 |
| 6 | 0.6021 | 16.24 | 6.2056 | 228.0 | σ | 18.15 | 14.2 | 11.85 | 11.72 |
| | | | | | ϵ | 247.7 | 182.7 | 182.7 | 181.4 |
| 8 | 1.0436 | 24.73 | 7.2777 | 135.9 | σ | 28.05 | 19.1 | 17.12 | 16.89 |
| | | | | | ϵ | 159.0 | 82.8 | 82.8 | 81.3 |

Table 8

| Degree of WAXS - Crystallinity of Samples | | | | | | |
|---|---|---|---|---|---|---|
| Test Run | | Sample | | | | |
| | Godet | 1 | 2 | 3 | 4 | 5 |
| Draw Ratio | | | | | | |
| 82A | 1 | 48.2 | 41.7 | 26.1 | 20.2 | 14.0 |
| | 2 | 44.6 | 37.0 | 24.6 | 17.1 | 19.0 |
| 109 B | 1 | 47.8 | 42.8 | 36.9 | 34.6 | 27.8 |
| | 2 | 44.4 | 37.6 | 26.6 | 19.0 | 16.7 |
| 109C | 2 | 53.9 | 50.7 | 41.3 | 36.2 | 30.9 |
| 109 D[3)] | 1 | 46.6 | 45.5 | 38.2 | 30.7 | 27.2 |
| 109 F | 1 | 57.4 | 57.6 | 57.5 | 57.4 | 56.5 |
| | 2 | 56.1 | 52.9 | 43.2 | 35.4 | 27.0 |
| 127 | 1 | 42.2 | - | 36.6 | 28.8 | 21.2 |
| | 2 | 45.2 | - | 30.6 | 27.7 | 18.0 |
| 178 A | 1 | 37.4 | 33.5 | 29.4 | 28.8 | 18.2 |
| | 2 | 34.3 | 28.8 | 24.6 | 19.4 | 15.8 |
| 178 D2 | 1 | 36.9 | 35.5 | 34.2 | 33.6 | 28.9 |
| | 2 | 35.6 | 34.6 | 29.8 | 24.2 | 19.5 |
| 179 A | 1 | 55.4 | 55.8 | 55.7 | 55.2 | 55.2 |
| | 2 | 54.5 | 51.7 | 45.5 | 37.7 | 32.0 |
| 179 D | 1 | 55.2 | - | 55.7 | - | - |
| | 2 | 55.5 | - | 45.4 | - | - |

3) Comparative example - no stabilizing agent

D.F. Beaumont, K.R. Randall
Nonwovens World, 1986, 76-80

/1/ USP no/ patent year
3.560.326/1971; 3.620.903/1971; 4.085.485/1978; 4.144.370/1979; 4.152.480/1979; 4.188.690/1980; 4.329.763/1982; 4.442.161/1984; 4.476.186/1984; 4.556.601/1985; 4.623.575/1986; 4.647.490/1987; 4.665.597/1987; 4.741.075/1988; 4.808.467/1989; 4.810.568/1989

/2/ Bela von Falkai
Syntesefasern, Verlag Chemie 1981
A. Ziabicki
Fundamentals of Fibre Formation, John Wiley&Sons, 1976
A. Ziabicki, H. Kawai
High-Speed Fiber Spinning, John Wiley & Sons, 1985

/3/ R.F. Becker, D.J. Carlsson, J.M. Cooke, S. Chmela
Polym. Deg. and Stab., 22, 1988, 313-323

/4/ R.L. Clough, K.T. Gillen
J. Pol. Sci., 19, 1981, 2041-2051

/5/ Encyclopedia of Polymer Science and Engineering
Vol. 4., 1990, 630, John Wiley & Sons

/6/ M. Ahmed
Polypropylene Fibers - science and technology, Elsevier, 1982, 38-82

/7/ GB 1.050.802/1966, USP 4.110.185/1978, USP 4.274.932/1981, USP 4.467.065/1984, USP 4.563.259/1986

/8/ T.S. Dunn, J.L. Williams
J. Ind. Irrad. Tech., 1, 1983, 33-49

/9/ Ye Yougcheng
Polym. Deg. and Stab., 37, 1992, 11-17
P. Gijsman, J. Hennekeus, D. Tummers
Polym. Deg. and Stab., 39, 1993, 225-233

/10/ H.H.G. Jellinek
Aspects of Degradation and Stabilization of Polymers, Elsevier 1978, 139-143

/11/ H.H.G. Jellinek
Degradation and Stabilization of Polymers, Elsevier, 1983, 20-36, 279

/12/ S.G. Kiryushkin, Yu. A. Shlyapnikov
Polym. Deg. and Stab., 1, 1989, 185-192

/13/ D.J. Carlsson, A. Garton, D.M. Wiles
Stab. and Deg. of Polym., 1, 1979, 56-67

/14/ J. Crank, G.S. Park
Diffusion in Polymers, Academic Press 1968, 45-64

/15/ J.Y. Moisan
in J. Comyn: Polymer Permeability, Elsevier ASP 1985, 119-176

/16/ P. Holden, G.A. Orchard, I.M. Ward
J. Polymer Sci., Polym. Phys. Ed., 23, 1985, 709
A.K. Taraiya, G.A.J. Ovchard, I.M. Ward
J. Appl. Polym. Sci., 41, 1990, 1659-1671

/17/ M. Mucha
Colloid & Polymer Sci., 264, 1986, 113-116

/18/ F. Bueche, W.M. Cashin, P. Debye
J. Chem. Phys., 20, 1952, 1956-1958
J. E. Tanner, K-J. Liu, J.E. Anderson
Macromolecules, 4, 1971, 586-588
B.A. Smith, E.T. Samulski, L-P. Yu, M.A. Winnik
Macromolecules, 18, 1985, 1901-1905
P.T. Gilmore, R. Falabella, R.L. Laurence
Macromolecules, 13, 1980, 880-883

/19/ J. Malik, A. Hrivik, E. Tomova

Polym. Deg. and Stab., 35, 1992, 61-66
N.C. Billingham, P.D. Calvert, I.W. Okopi, A. Uzuner
Polym. Deg. and Stab., 31, 1991, 23-36

/20/ F. Gugumus
Polym. Deg. and Stab., 40, 1993, 167-215

## Claims

1. A process for the production of gamma-radiation resistant polypropylene fibre suitable for the production of a radiation sterilizable polypropylene non-woven fabric, which process comprises the stages of melt spinning, drawing, finishing, crimping and drying and optionally gamma-irradiating a polypropylene polymer base-stabilized with a peroxide degrading agent and of fibre quality, characterized by:

   (a) in a melt spinning stage, controlling the solidification and crystallization of the spinning melt by means of the cooling rate of the melt and the tension of the fibre cable undergoing solidification, so as to obtain a spinning product having, while avoiding a paracrystalline or smectic phase, a structural system with a spherulitic superstructure and a monoclinic-amorphous microstructure, the crystallinity of which is lower than the maximum crystallinity of the polymer and having a crystallinity value higher than 15 mass-%, and an average chain orientation of less than value 0.6 (60%) based on birefringence measurement;
   (b) in a solid state drawing stage subsequent to the melt spinning, controlling the super- and microstructure of the polymer of the draw product by regulating the draw ratio and the chain orientation, so as to maintain, while avoiding a microfibrillar structure, the structure of deformed spherulites in the superstructure, and the monoclinic-amorphous system in the microstructure, the crystallinity value being equal to or lower than that of the spinning product and the average orientation being less than 0.76 (76%) based on birefringence measurement
   (c) using, in all fibre production stages following the melt spinning and especially in the drawing and drying stages, a temperature not exceeding 120°C for the polymer;
   (d) additionally if the fibre is produced at a temperature of 80°C or less stabilizing the fibre against thermal changes by regulating the draw ratio in the drawing stage and, if an irradiation step is present, regulating the gamma-radiation dose;
   (e) regulating the tensile strength at yield limit of the product fibre by means of the molecular weight and weight distribution of the polymer, and the draw ratio in the fibre drawing stage; the weight average molecular weight of the polypropylene of fibre quality being between $1.5 \times 10^5$ and $2.5 \times 10^5$, and the molecular weight distribution between 2 and 6;
   (f) when the polymer has a high average chain orientation of 0.6 to 0.76, especially close to the area of microfibrillar transition, using at least one radical scavenger or energy extinguisher as a stabilization agent in addition to the basic stabilization.

2. The process according to claim 1, characterized in that the highest actual temperature of the polymer is 100°C.

3. The process according to any one of the preceding claims, characterized in that in accordance with paragraph (d) of claim 1 if a gamma-radiation dose is used for stabilizing the thermally unstabilized product fibre, the minimum radiation dose is 2.5 Mrad.

4. The process according to any one of the preceding claims, characterized in that if the fibre is produced at a temperature of 80°C or less the elongation of the fibre is regulated by means of the spinning melt temperature, drawing temperature, draw ratio, the molecular weight and weight distribution of the polymer, and radiation dose.

5. A process according to claim 1, in which in all fibre production stages following the melt spinning as low as possible temperature is used for the polymer.

6. The process according to the claims 1, 3, 4 or 5, characterized in that a temperature of 25 to 80°C is used.

7. The process according to any one of the preceding claims, characterized in that the draw ratio used in the drawing stage is adjusted to be from 1.25 to 4.00.

8. The process according to claim 4, characterized in that the fibre is irradiated with a gamma-radiation dose of from 2.5 to 5.0 Mrad, so that the thermo mechanical contraction of the product fibre, at applied measuring stress of 0.5 mN/tex, remains below the value of 25% calculated from the elongation of an non-irradiated fibre, and simultaneously the strength values of the product fibre remain above the pre- and post-radiation yield limits.

9. The process according to any one of the preceding claims, characterized in that the gauge of the spinning and draw fibres is from 8.5 to 1.5 dtex.

10. The process according to the claim 1, characterized in that the fibre polymer is base-stabilized with a phosphite-phosphonite-mixture, known as a peroxide degrading agent, and that the additional stabilization agent is a hindered amine, known as a radical terminator or chromophore energy extinguisher, especially a 2,2,4,4-tetramethyl-piperidyl derivative, or mixture thereof.

11. The process according to the claim 10, characterized in that one or more hindered amines are added to the polymer matrix, as additional stabilizer(s) in an amount sufficient for the supersaturation of the amorphous portion of the polymer.

12. The process of any one of the preceding claims in which the total amount of stabilizing agents present in the final fibre is less than 0.5% by weight of the fibre material.

13. Use of a polypropylene fibre made according to any one of the claims 1 to 12 for the production of a gamma-sterilizable non-woven fabric.

14. Use of a polypropylene fibre produced according to any one of claims 1 to 12, optionally in combination with other fibres, in the production of a mechanically, hydraulically or thermally bonded non-woven fabric.

15. A process for the production of a non-woven fabric, characterized in that fibres are made according to any one of the claims 1 to 12, they are formed into a web, which web is mechanically, thermally or hydraulically bonded to form a non-woven fabric, which optionally is sterilized by radiation.

16. Process according to the claim 15, characterized in that in the production and treatment of the fabric, the highest allowed actual temperature of the polymer is 120°C.

17. Process according to the claim 15, characterized in that the fabric elongation is regulated by regulating the fibre elongation and, if used, the radiation dose, the total radiation dose exceeding a dose of 1.5 Mrad and the fibre elongation exceeding the pre- and post-radiation yield limit.

18. Process according to the claim 15, characterized in that the fabric strength is increased by increasing the chain length of the polymer and the yield strength and yield energy of the fibre.

19. Process according to the claim 15, characterized in that, if the fabric is subjected to radiation, additional stabilization of the fabric against thermal changes is carried out by regulating the radiation dose in a gamma-radiation sterilization.

**Patentansprüche**

1. Verfahren zur Herstellung einer gammastrahlungsbeständigen Polypropylenfaser, die zur Herstellung eines mit Strahlung sterilisierbaren Polypropylen-Vliesstoffs geeignet ist, wobei bei dem Verfahren folgende Stufen durchlaufen werden: das Schmelzspinnen, Ziehen, Ausrüsten, Kräuseln und Trocknen und gegebenenfalls Gammabestrahlung eines Polypropylen-Polymers, das mittels eines Peroxidzerlegungsmittels basisstabilisiert und von Faserqualität ist, dadurch gekennzeichnet, daß

   a) in einer Schmelzspinnstufe das Aushärten und Kristallisieren der Spinnschmelze durch die Abkühlungsgeschwindigkeit der Schmelze und die Zugspannung des Faserkabels, während es aushärtet, so gesteuert wird, daß sich ein Spinnprodukt ergibt, das, unter Vermeidung einer parakristallinen oder smektischen Phase, ein strukturiertes System mit einer sphärolitischen Superstruktur und eine monoklinamorphe Mikrostruktur aufweist, deren Kristallinität kleiner als die maximale Kristallinität des Polymers ist und einen Kristallinitätsgrad

von mehr als 15 Massen-% sowie eine mittlere Kettenorientierung von weniger als 0,6 (60%) auf der Basis einer Doppelbrechungsmessung aufweist;

b) in einer Ziehstufe im festen Zustand nach dem Schmelzspinnen die Super- und Mikrostruktur des Polymers des gezogenen Produkts durch Regelung des Zieverhältnisses und der Kettenorientierung so gesteuert wird, daß, unter Vermeidung einer mikrofaserartigen Struktur, die Struktur der verformten Sphärulite in der Superstruktur und das monoklinamorphe System in der Mikrostruktur erhalten bleiben, wobei der Kristallinitätswert gleich dem oder kleiner als der des Spinnprodukts und die mittlere Orientierung kleiner als 0,76 (76%) auf der Basis einer Doppelbrechungsmessung ist,

c) in allen Faserherstellungsstufen nach dem Schmelzspinnen und insbesondere in den Zieh- und Trocknungsstufen eine Temperatur für das Polymer angewandt wird, die 120°C nicht überschreitet;

d) die Faser zusätzlich, wenn sie bei einer Temperatur von 80°C oder weniger hergestellt worden ist, gegen thermische Änderungen durch Regelung des Ziehverhältnisses in der Ziehstufe und, wenn ein Bestrahlungsschritt vorliegt, durch Regelung der Gammastrahlungsdosis stabilisiert wird;

e) die Zugfestigkeit an der Fließgrenze der erzeugten Faser mittels des Molekulargewichts und der Gewichtsverteilung des Polymers und des Ziehverhältnisses in der Faserziehstufe geregelt wird; wobei das mittlere Molekulargewicht des Polypropylens mit Faserqualität zwischen 1,5 x $10^5$ und 2,5 x $10^5$ und die Molekulargewichtsverteilung zwischen 2 und 6 liegt;

f) wenn das Polymer eine hohe mittlere Kettenorientierung von 0,6 bis 0,76, insbesondere in der Nähe des Bereichs des mikrofaserartigen Übergangs, aufweist, wenigstens ein Radikalspülmittel oder Energievernichtungsmittel als Stabilisierungsmittel, zusätzlich zur Basisstabilisierung, angewandt wird.

**2.** Verfahren nach Anspruch 1, dadurch gekennzeichnet, daß die höchste Ist-Temperatur des Polymers 100°C beträgt.

**3.** Verfahren nach einem der vorstehenden Ansprüche, dadurch gekennzeichnet, daß nach Absatz d) des Anspruchs 1, wenn eine Gammastrahlungsdosis zur Stabilisierung der thermisch unstabilisierten erzeugten Faser angewandt wird, die minimale Strahlungsdosis 2,5 Mrad beträgt.

**4.** Verfahren nach einem der vorstehenden Ansprüche, dadurch gekennzeichnet, daß, wenn die Faser bei einer Temperatur von 80°C oder weniger hergestellt wird, die Dehnung der Faser mittels der Spinnschmelztemperatur, der Ziehtemperatur, des Ziehverhältnisses, des Molekulargewichts und der Gewichtsverteilung des Polymers und der Strahlungsdosis geregelt wird.

**5.** Verfahren nach Anspruch 1, bei dem in allen Faserherstellungsstufen nach dem Schmelzspinnen eine möglichst niedrige Temperatur für das Polymer angewandt wird.

**6.** Verfahren nach den Ansprüchen 1, 3, 4 oder 5, dadurch gekennzeichnet, daß eine Temperatur von 25 bis 80°C angewandt wird.

**7.** Verfahren nach einem der vorstehenden Ansprüche, dadurch gekennzeichnet, daß das Ziehverhältnis, das in der Ziehstufe angewandt wird, im Bereich vom 1,25 bis 4,00 liegt.

**8.** Verfahren nach Anspruch 4, dadurch gekennzeichnet, daß die Faser mit einer Gammastrahlungsdosis von 2,5 bis 5,0 Mrad bestrahlt wird, so daß die thermomechanische Kontraktion der erzeugten Faser, bei Anwendung einer Meßbelastung von 0,5 mN/tex, unter dem anhand der Dehnung einer nichtbestrahlten Faser berechneten Wert von 25% bleibt und gleichzeitig die Festigkeitswerte der erzeugten Faser über den Vor- und Nachbestrahlungs-Fließgrenzen bleiben.

**9.** Verfahren nach einem der vorstehenden Ansprüche, dadurch gekennzeichnet, daß die Stärke der Spinn- und Ziehfaser im Bereich von 8,5 bis 1,5 dtex liegt.

**10.** Verfahren nach Anspruch 1, dadurch gekennzeichnet, daß das Faserpolymer mittels eines Phosphit-Phosphonit-Gemisches, das als Peroxidzerlegungsmittel bekannt ist, basisstabilisiert wird und daß das zusätzliche Stabilisierungsmittel ein gehindertes Amin ist, das als Radikal-Terminator oder chromophorer Energievernichter bekannt ist, insbesondere ein 2,2,4,4-Tetramethyl-Piperidyl-Derivat oder Gemisch daraus.

**11.** Verfahren nach Anspruch 10, dadurch gekennzeichnet, daß ein oder mehrere gehinderte Amine der Polymermatrix zugesetzt werden, und zwar als zusätzlicher bzw. zusätzliche Stabilisator(en), in einer Menge, die zur Übersättigung des amorphen Teils des Polymers ausreichend ist.

**12.** Verfahren nach einem der vorstehenden Ansprüche, bei dem die Gesamtmenge der in der fertigen Faser vorhandenen Stabilisierungsmittel weniger als 0,5 Gew.-% des Fasermaterials beträgt.

**13.** Anwendung der nach einem der Ansprüche 1 bis 12 hergestellten Polypropylenfaser zur Herstellung eines durch Gammastrahlung sterilisierbaren Vliesstoffs.

**14.** Anwendung einer nach einem der Ansprüche 1 bis 12 hergestellten Polypropylenfaser, gegebenenfalls in Kombination mit anderen Fasern, zur Herstellung eines mechanisch, hydraulisch oder thermisch verbundenen Vliesstoffs.

**15.** Verfahren zur Herstellung eines Vliesstoffs, dadurch gekennzeichnet, daß nach einem der Ansprüche 1 bis 12 hergestellte Fasern zu einer Bahn geformt werden, die mechanisch, thermisch oder hydraulisch zur Bildung eines Vliesstoffs verbunden wird, der gegebenenfalls durch Bestrahlung sterilisiert wird.

**16.** Verfahren nach Anspruch 15, dadurch gekennzeichnet, daß bei der Herstellung und Behandlung des Vliesstoffs die höchste zulässige Ist-Temperatur des Polymers 120°C beträgt.

**17.** Verfahren nach Anspruch 15, dadurch gekennzeichnet, daß die Vliesstoffdehnung durch Regelung der Faserdehnung und, bei Anwendung einer Bestrahlung, die Bestrahlungsdosis geregelt wird, wobei die Gesamtbestrahlungsdosis eine Dosis von 1,5 Mrad überschreitet und die Faserdehnung die Vor- und Nachbestrahlungs-Fließqrenze überschreitet.

**18.** Verfahren nach Anspruch 15, dadurch gekennzeichnet, daß die Vliesstofffestigkeit durch Erhöhung der Kettenlänge des Polymers und der Fließfestigkeit sowie der Fließenergie der Faser gesteigert wird.

**19.** Verfahren nach Anspruch 15, dadurch gekennzeichnet, daß bei Bestrahlung des Vliesstoffs eine zusätzliche Stabilisierung des Vliesstoffs gegen thermische Änderungen durch Regelung der Bestrahlungsdosis bei einer Gammabestrahlungssterilisation bewirkt wird.

## Revendications

**1.** Procédé de production d'une fibre de polypropylène résistant aux rayonnements gamma qui convient pour la production d'un tissu non tissé en polypropylène stérilisable par rayonnement, lequel procédé comprend les étapes de filage en fusion, d'étirage, de finissage, de frisage et de séchage et éventuellement d'irradiation gamma d'un polymère de polypropylène stabilisé basiquement avec un agent dégradant les peroxydes et de qualité fibre, caractérisé en ce que :

(a) dans une étape de filage en fusion, la solidification et la cristallisation de la masse fondue de filage sont contrôlées au moyen de la vitesse de refroidissement de la masse fondue et de la tension du câble de fibres qui subit la solidification de manière à obtenir un produit de filage ayant, tout en évitant une phase paracristalline ou smectique, un système structural avec une superstructure sphérulitique et une microstructure monoclinique-amorphe, dont la cristallinité est inférieure à la cristallinité maximale du polymère et ayant une valeur de cristallinité inférieure à 15 % en masse, et une orientation de chaîne moyenne inférieure à 0,6 (60 %) d'après une mesure de biréfringence;
(b) dans une étape d'étirage à l'état solide qui suit le filage en fusion, la super- et microstructure du polymère du produit étiré sont contrôlées par régulation du rapport d'étirage et de l'orientation des chaînes de manière à maintenir, tout en évitant une structure microfibrillaire, la structure des sphérulites déformés dans la superstructure, et le système monoclinique-amorphe dans la microstructure, la valeur de cristallinité étant égale ou inférieure à celle du produit de filage et l'orientation moyenne étant inférieure à 0,76 (76 %) d'après une mesure de biréfringence ;
(c) une températures ne dépassant pas 120°C pour le polymère est utilisée dans toutes les étapes de production de fibres après le filage en fusion et en particulier dans les étapes d'étirage et de séchage ;
(d) en outre, si la fibre est produite à une température de 80°C ou moins, la fibre est stabilisée contre les variations thermiques par régulation du rapport d'étirage dans l'étape d'étirage et, si une étape d'irradiation est présente, par régulation de la dose de rayonnement gamma ;

(e) la résistance à la traction à la limite élastique de la fibre produite est régulée au moyen de la masse moléculaire et de la distribution de masse du polymère, et le rapport d'étirage est régulé dans l'étape d'étirage des fibres; la masse moléculaire moyenne en poids du polypropylène de qualité fibre étant comprise entre 1,5 x $10^5$ et 2,5 x $10^5$, et la distribution de masse moléculaire étant comprise entre 2 et 6 ;
(f) lorsque le polymère a une haute orientation moyenne des chaînes de 0,6 à 0,76, en particulier proche de la zone de transition microfibrillaire, au moins un piégeur de radicaux ou un agent absorbant l'énergie est utilisé comme agent de stabilisation en plus de la stabilisation basique.

**2.** Procédé selon la revendication 1, caractérisé en ce que la plus haute température réelle du polymère est 100 °C.

**3.** Procédé selon l'une quelconque des revendications précédentes, caractérisé en ce que, selon le paragraphe (d) de la revendication 1, si une dose de rayonnement gamma est utilisée pour stabiliser la fibre produite non stabilisée thermiquement, la dose de rayonnement minimum est de 2,5 Mrad.

**4.** Procédé selon l'une quelconque des revendications précédentes, caractérisé en ce que si la fibre est produite à une température de 80°C ou moins, l'allongement de la fibre est régulé au moyen de la température de la masse fondue de filage, de la température d'étirage, du rapport d'étirage, de la masse moléculaire et de la distribution de masse du polymère, et de la dose de rayonnement.

**5.** Procédé selon la revendication 1, dans lequel dans toutes les étapes de production de fibres qui suivent le filage en fusion on utilise une température la plus basse possible pour le polymère.

**6.** Procédé selon les revendications 1, 3, 4 ou 5, caractérisé en ce que l'on utilise une température de 25 à 80°C.

**7.** Procédé selon l'une quelconque des revendications précédentes, caractérisé en ce que le rapport d'étirage utilisé dans l'étape d'étirage est ajusté pour être compris entre 1,25 et 4,00.

**8.** Procédé selon la revendication 4, caractérisé en ce que la fibre est irradiée avec une dose de rayonnement gamma de 2,5 à 5,0 Mrad de sorte que la contraction thermomécanique de la fibre produite, à une contrainte de mesure appliquée de 0,5 mN/tex, reste inférieure à la valeur de 25 % calculée d'après l'allongement d'une fibre non irradiée, et simultanément les valeurs de résistance à la traction de la fibre produite restent supérieures aux limites élastiques avant et après irradiation.

**9.** Procédé selon l'une quelconque des revendications précédentes, caractérisé en ce que la finesse des fibres de filage et d'étirage est de 8,5 à 1,5 dtex.

**10.** Procédé selon la revendication 1, caractérisé en ce que le polymère des fibres est stabilisé basiquement avec un mélange phosphite-phosphonite, connu comme étant un agent dégradant les peroxydes, et en ce que l'agent de stabilisation supplémentaire est une amine à empêchement stérique, connue comme étant un terminateur de radicaux ou un extincteur d'énergie chromophore, en particulier un dérivé de 2,2,4,4-tétraméthylpipéridyle, ou un mélange de ceux-ci.

**11.** Procédé selon la revendication 10, caractérisé en ce qu'une ou plusieurs amines à empêchement stérique sont ajoutées à la matrice de polymère, en tant que stabilisant(s) supplémentaire(s) en une quantité suffisante pour la sursaturation de la partie amorphe du polymère.

**12.** Procédé selon l'une quelconque des revendications précédentes, dans lequel la quantité totale d'agents stabilisants présents dans la fibre finale est inférieure à 0,5 % en masse de la matière de la fibre.

**13.** Utilisation d'une fibre de polypropylène produite selon l'une quelconque des revendications 1 à 12 pour la production d'un tissu non tissé stérilisable aux rayons gamma.

**14.** Utilisation d'une fibre de polypropylène produite selon l'une quelconque des revendications 1 à 12, éventuellement en combinaison avec d'autres fibres, dans la production d'un tissu non tissé lié mécaniquement, hydrauliquement ou thermiquement.

**15.** Procédé pour la production d'un tissu non tissé, caractérisé en ce que des fibres sont produites selon l'une quelconque des revendications 1 à 12, sont mises sont forme d'une nappe, laquelle nappe est liée mécaniquement,

thermiquement ou hydrauliquement pour former un tissu non tissé qui est éventuellement stérilisé par rayonnement.

**16.** Procédé selon la revendication 15, caractérisé en ce que, dans la production et le traitement du tissu, la plus haute température réelle admise du polymère est de 120°C.

**17.** Procédé selon la revendication 15, caractérisé en ce que l'allongement du tissu est régulé par régulation de l'allongement des fibres et, si elle est utilisée, de la dose de rayonnement, la dose de rayonnement totale dépassant une dose de 1,5 Mrad et l'allongement des fibres dépassant la limite élastique avant et après irradiation.

**18.** Procédé selon la revendication 15, caractérisé en ce que la résistance à la traction du tissu est augmentée par augmentation de la longueur des chaînes du polymère et de la limite élastique et de l'énergie de limite élastique de la fibre.

**19.** Procédé selon la revendication 15, caractérisé en ce que, si le tissu est soumis à un rayonnement, une stabilisation supplémentaire du tissu contre les modifications thermiques est réalisée par régulation de la dose de rayonnement dans une stérilisation par rayonnement gamma.

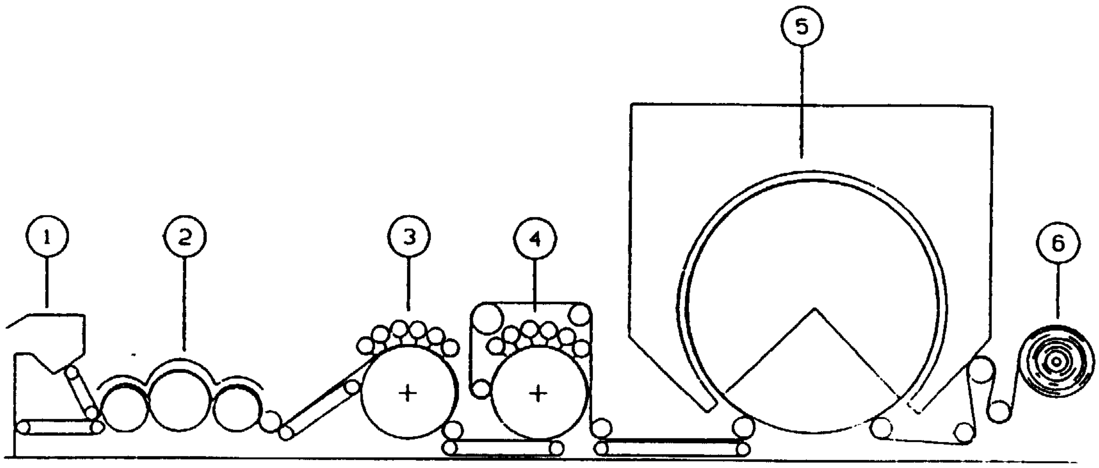


Fig.1. High-pressure Hydraulic Entanglement Production Line

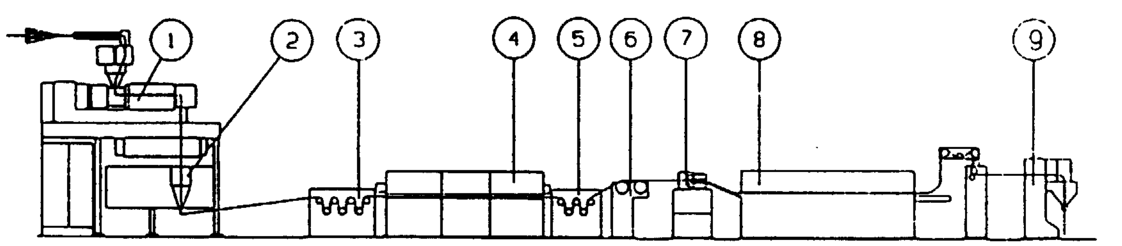


Fig.3. PP Staple Fibre Processing Line - pilot scale -

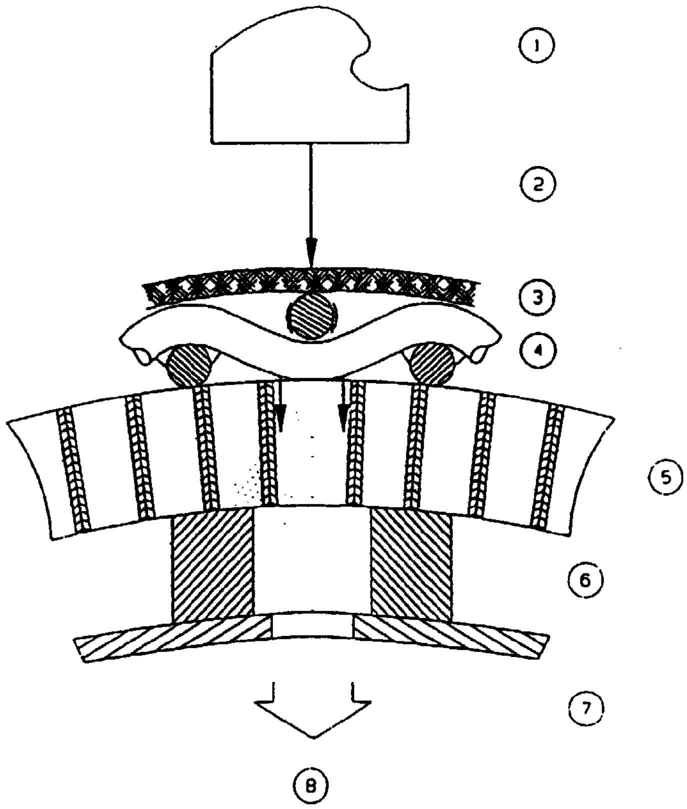


Fig.2. Water Jet Needling Station

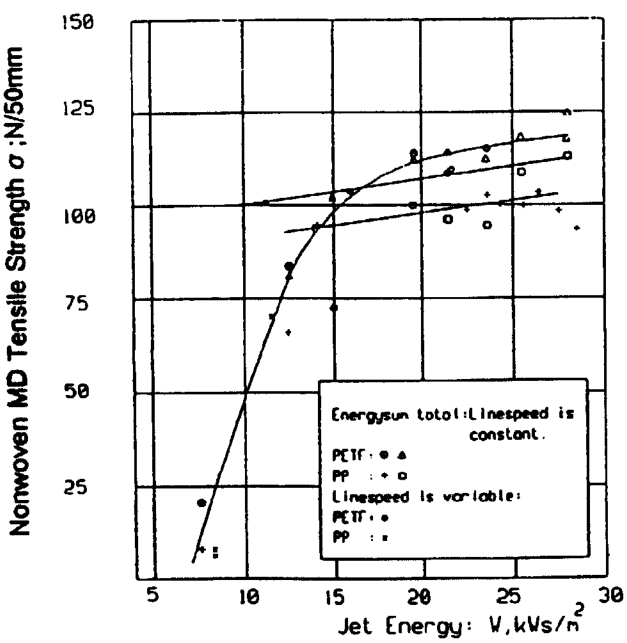


Fig.4. Nonwoven Tensile Strength as Function of Jet Energy

Fig.5. Nonwoven Tensile Strength as a Function of Yield Energy

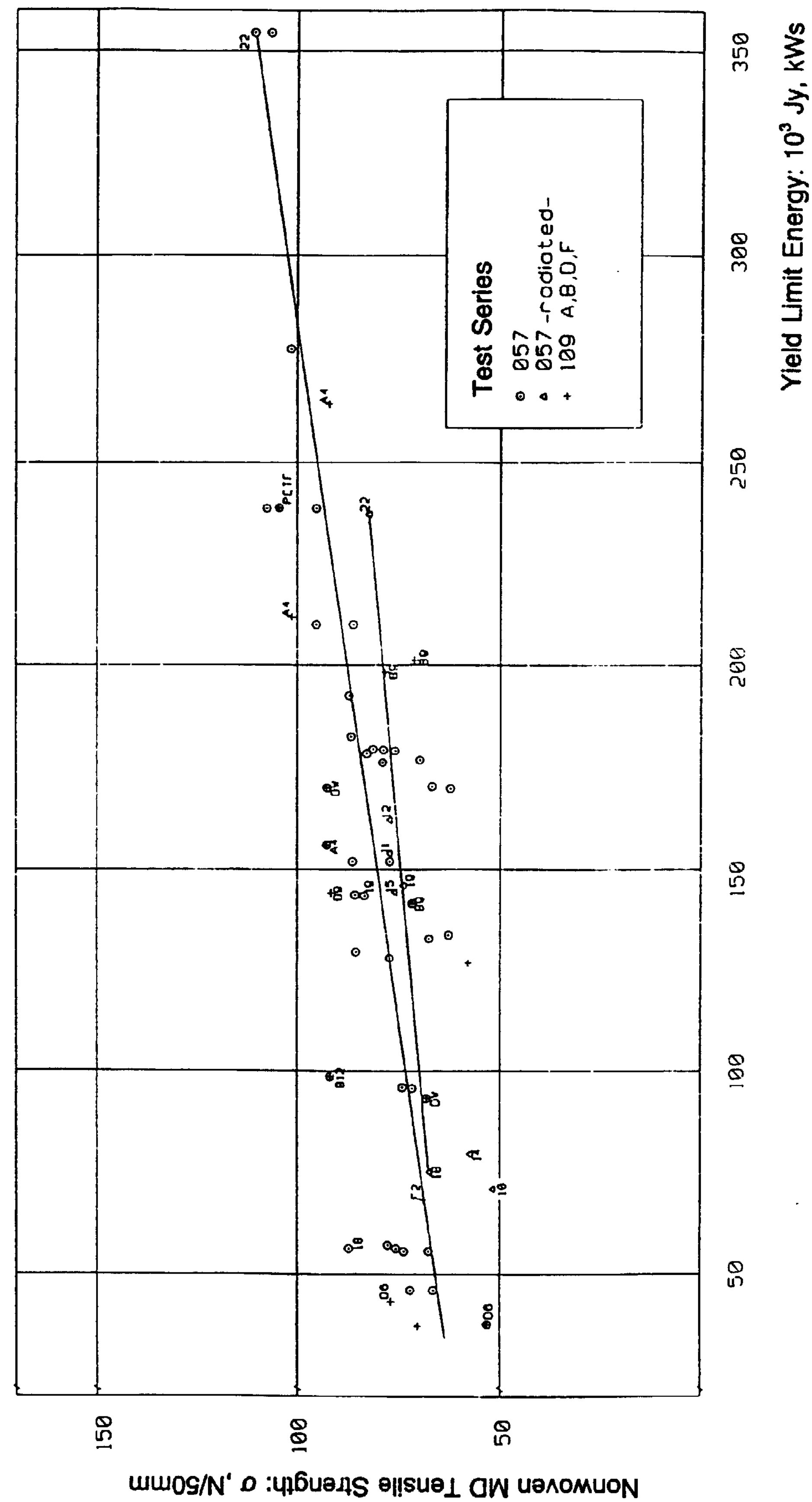

Fig.6. Stress-Strain-Curves for PP-Fibres

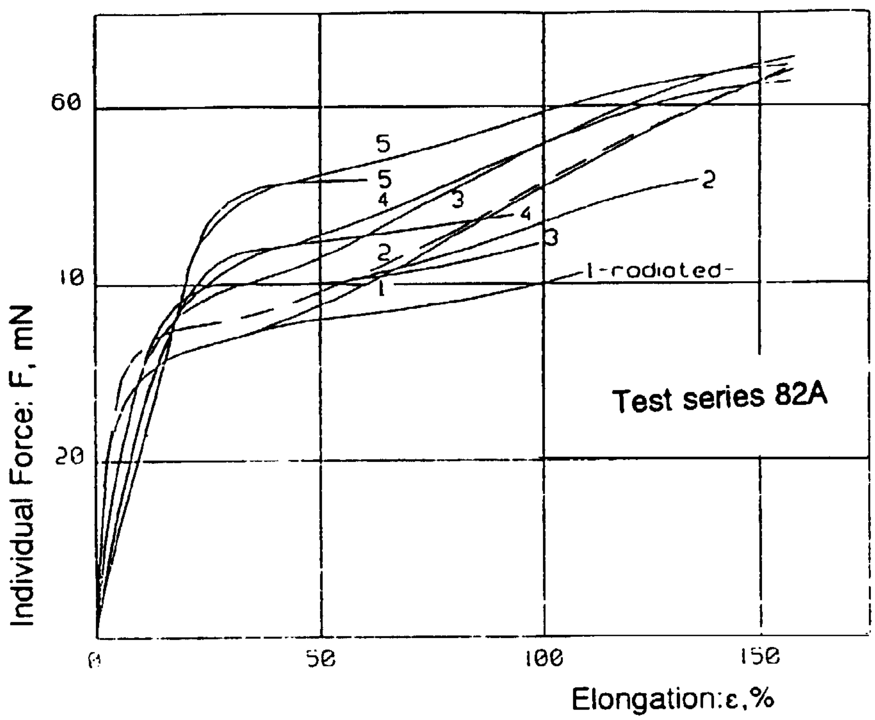


Fig.7. TMA-Curves for PP-Fibres

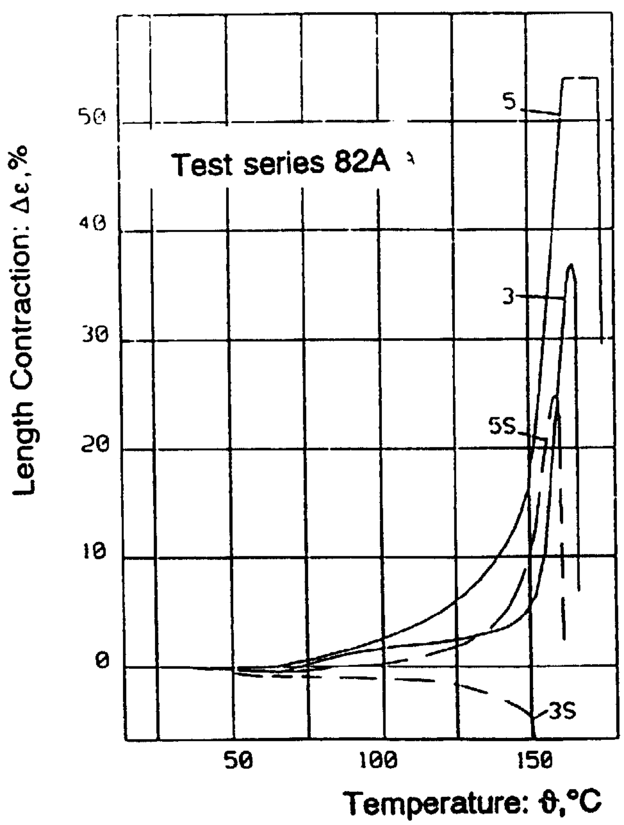

Fig.8. TMA-Curves for PP-Fibres

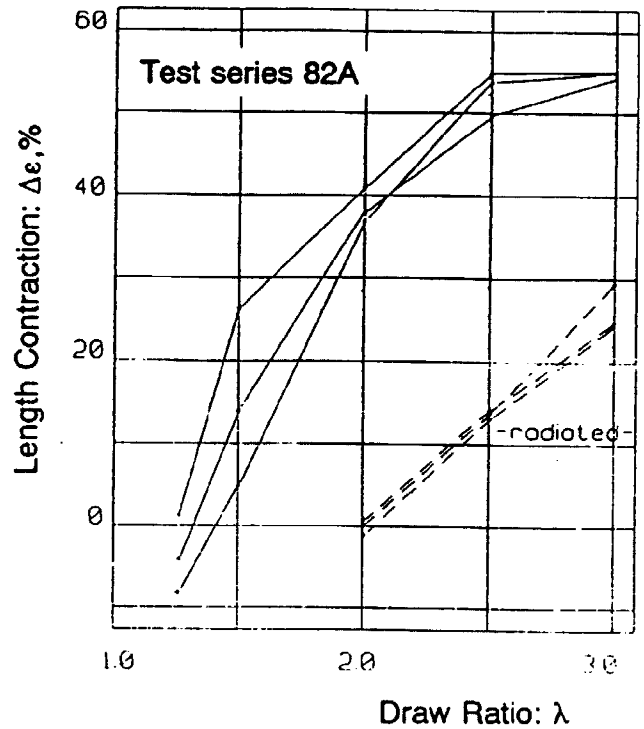


Fig.9. Elongation Versus Average Orientation

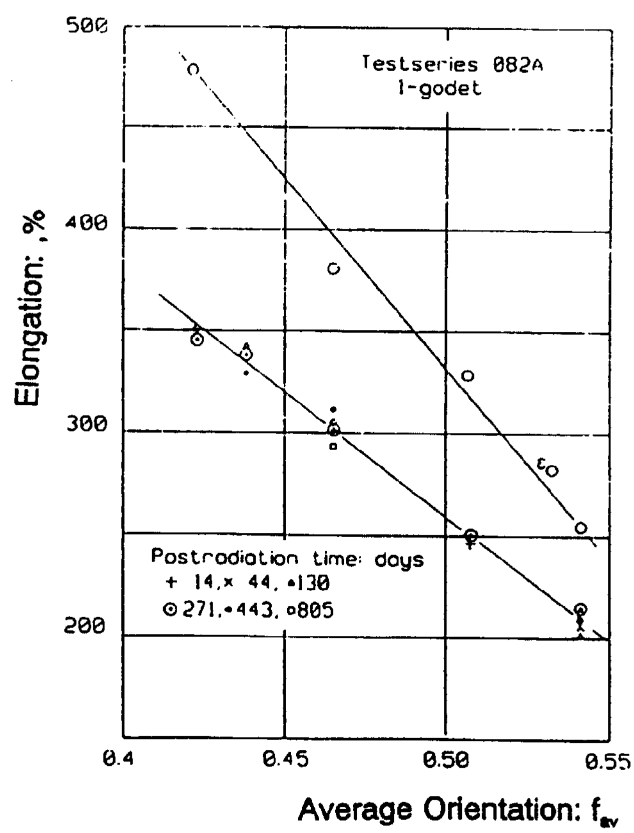

Fig.10. Elongation Versus Orientation

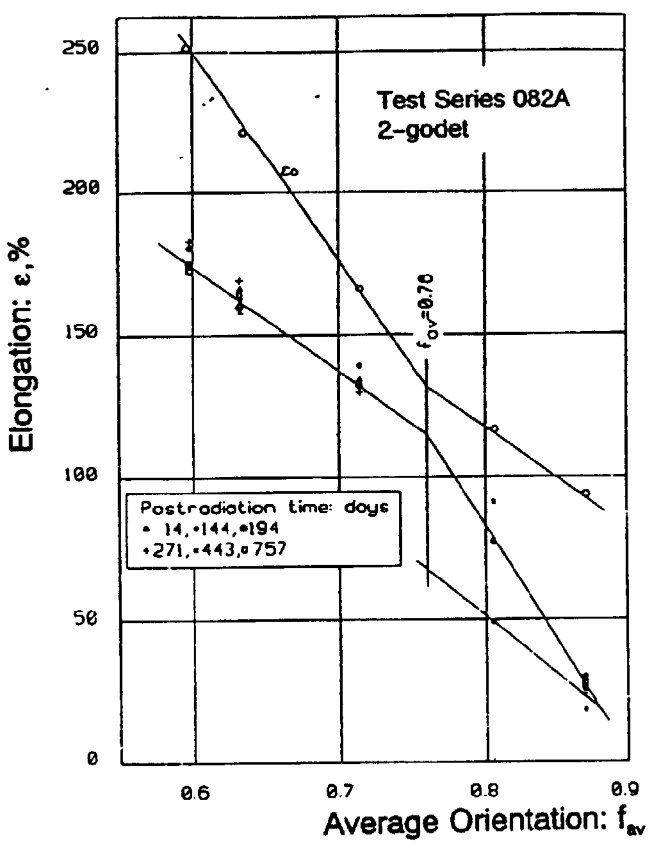


Fig.11. Radiation Damage Versus Orientation

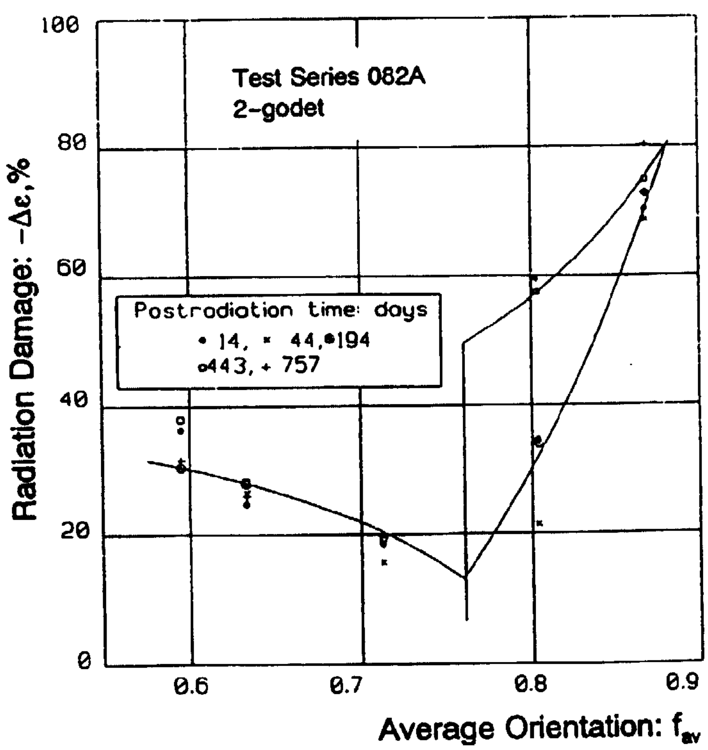

Fig.12. Stress-Strain-Curves for PP-Nonwovens and -fibres

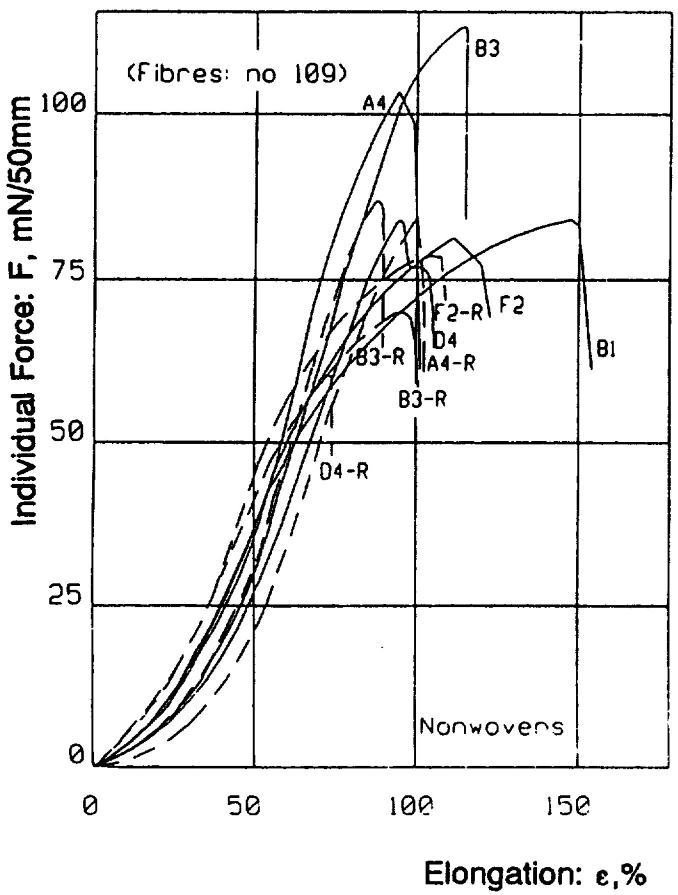


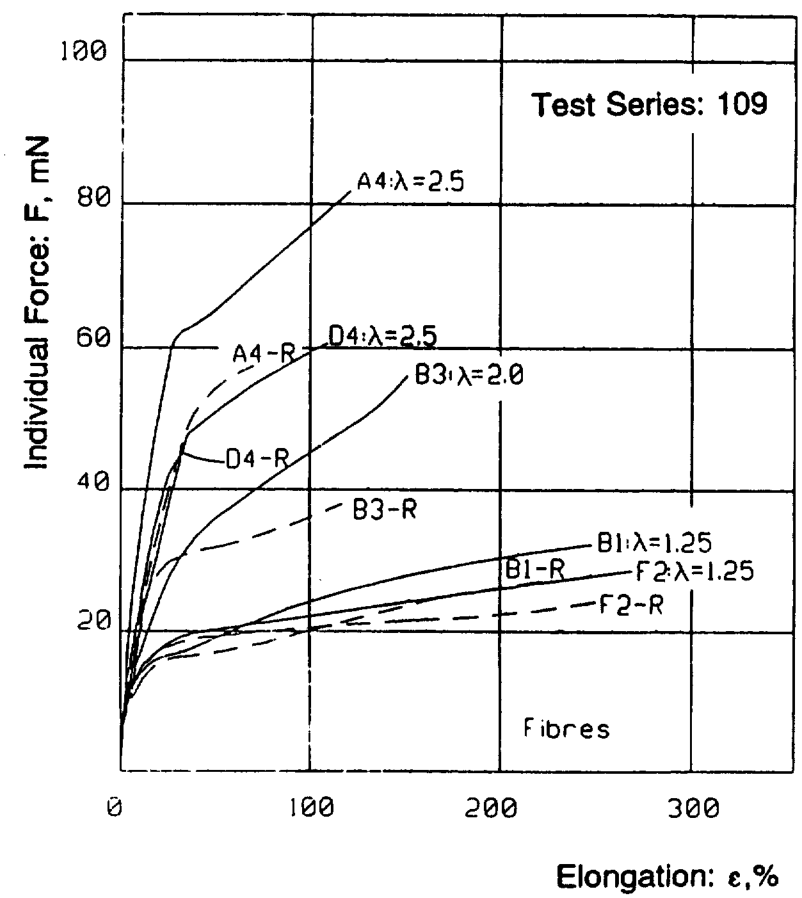

Fig.13 Cable Tension as a Function of Average Orientation
Radiation Damage as a Function of Average Orientation

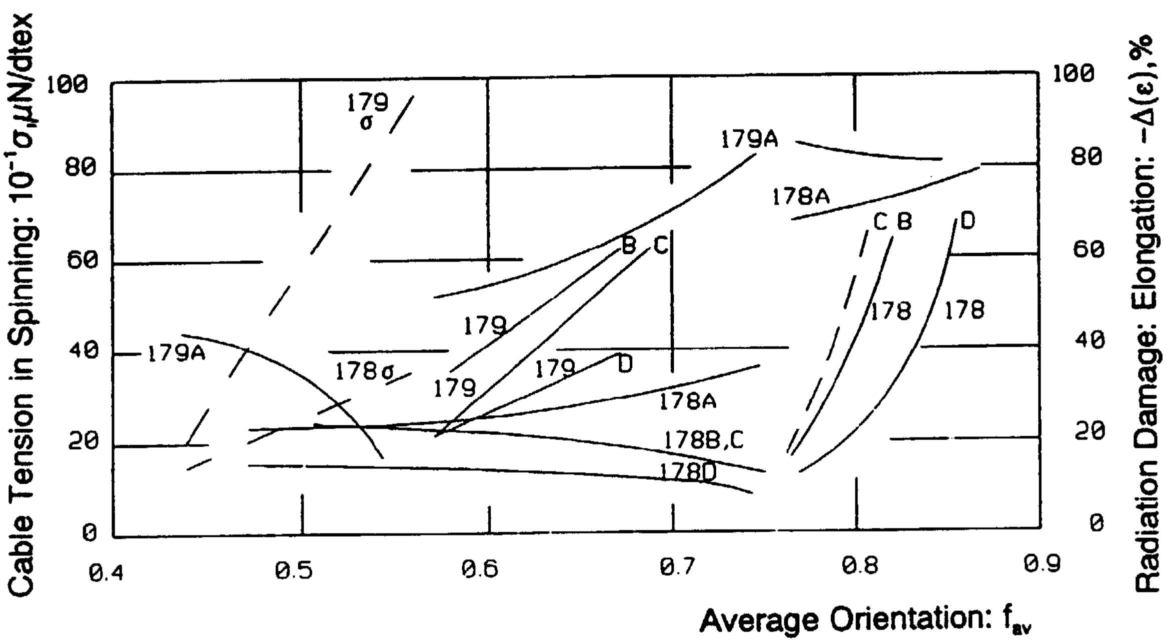


Fig.14. Radiation Damage as a Function of Average Orientation
WAXS-Crystallinity as a Function of Average Orientation

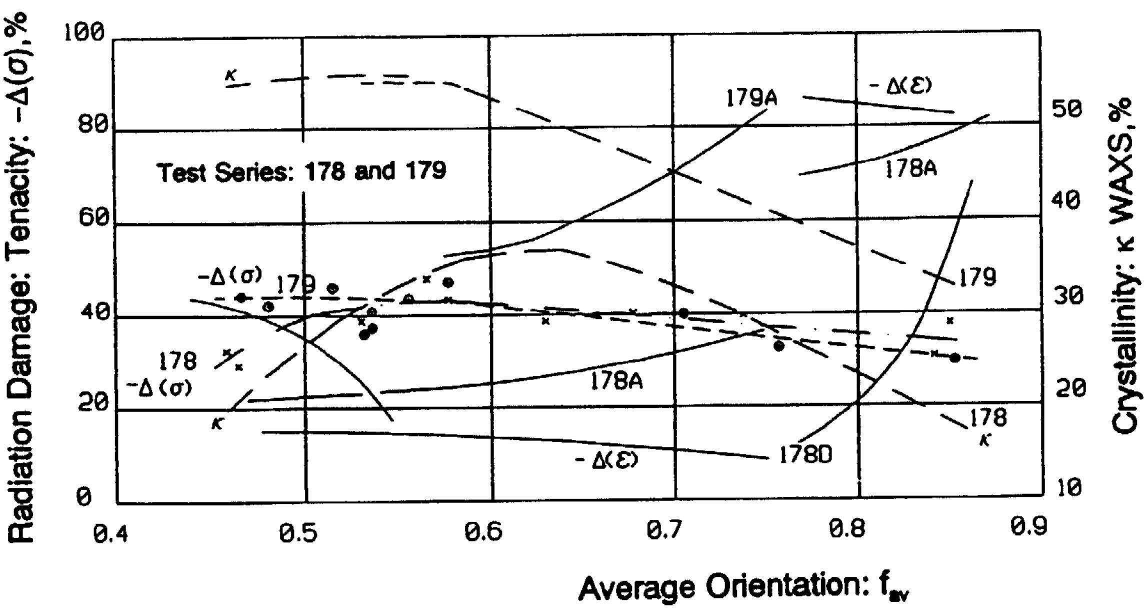

Fig.15. Radiation Damage as a Function of Average Orientation